# EUROPEAN PATENT APPLICATION

(11) **EP 4 840 950 A1**
(43) Date of publication of application: **18.11.2026**
(21) Application number: 25755236.4
(22) Date of filing: 13.02.2025
(51) Int. Cl.: C12N 9/18, C12N 15/70, A23K 20/189, A62D 3/02

(54) **FUMONISIN DEGRADING ENZYME AND USE THEREOF**

(30) Priority: 13.02.2024 KR 20240020459
(71) Applicant: CJ Cheiljedang Corporation, Seoul 04560 (KR)
(72) Inventor: KANG, Young Seo, Seoul 04560 (KR); YANG, Tae Joo, Seoul 04560 (KR); KIM, Nam Yoon, Seoul 04560 (KR); LEE, Seung-hee, Seoul 04560 (KR); JUNG, Gwanyoung, Seoul 04560 (KR); LEE, Whiso, Seoul 04560 (KR); PARK, Taeseo, Seoul 04560 (KR)
(74) Representative: Meissner Bolte Partnerschaft mbB
(86) International application number: PCT/KR2025/002137
(87) International publication number: WO 2025/174086

(57) **Abstract**

Provided are a fumonisin-degrading enzyme and use thereof.

## Description

### [Technical Field]

The present disclosure relates to a fumonisin-degrading enzyme and use thereof.

### [Background Art]

Fumonisin is a mycotoxin predominantly produced by *Fusarium verticillioides* and *Fusarium proliferatum* which are plant pathogenic fungi. These fungi often contaminate corn or corn-based products, and cause various diseases when ingested by humans or animals through foods or feeds. Generally, fumonisin is known to have hepatotoxicity and nephrotoxicity in animals, and is known to be related to esophageal cancer and neural tube defects in humans. For this reason, fumonisin B1, which is the most frequently found among fumonisins, is classified by the International Agency for Research on Cancer (IARC) as Group 2B, indicating it is possibly carcinogenic to humans.

Accordingly, there is a need for an effective composition or method for detoxifying fumonisin present in corn or corn-based products.

### [Prior Art Documents]

(Patent Document 1) WO2022-243722 A1

### [Disclosure]

### [Technical Problem]

The problem to be solved by the present disclosure relates to a fumonisin-degrading enzyme and use thereof.

### [Technical Solution]

An object of the present disclosure is to provide a variant polypeptide having fumonisin degradation activity.

Another object of the present disclosure is to provide a composition comprising the variant polypeptide.

Still another object of the present disclosure is to provide use of any one or more of the variant polypeptide and a host cell expressing the same for reaction with fumonisin.

Still another object of the present disclosure is to provide use of any one or more of the variant polypeptide and a host cell expressing the same for detoxifying fumonisin present in foods and/or feeds.

Still another object of the present disclosure is to provide a method of degrading fumonisin, the method comprising reacting fumonisin with any one or more of the variant polypeptide and a host cell expressing the variant polypeptide.

Still another object of the present disclosure is to provide a polynucleotide encoding the variant polypeptide.

Still another object of the present disclosure is to provide a host cell comprising any one or more of the variant polypeptide; and the polynucleotide encoding the same.

Still another object of the present disclosure is to provide a method of preparing the variant polypeptide.

### [Advantageous Effects]

A mycotoxin, fumonisin may be effectively degraded by using the polypeptide of the present disclosure, which has fumonisin degradation activity.

### [Brief Description of the Drawing]

FIG. 1 shows the relative activities of the fumonisin-degrading enzyme CB1 (SEQ ID NO: 1) and variants thereof, M1 to M7;
FIG. 2 shows the relative activities of fumonisin-degrading enzyme variants, M3 and M8 to M12;
FIG. 3 shows the relative activities of fumonisin-degrading enzyme variants, M3 and M13 to M16;
FIG. 4 shows the relative activities of fumonisin-degrading enzyme variants, M15 and M17 to M21; and
FIG. 5 shows the relative activities of the fumonisin-degrading enzyme variant M17 and variants thereof M22 to M23.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present disclosure.

Further, a number of papers and patent documents are referenced and cited throughout this specification. The disclosures of the cited papers and patent documents are incorporated herein by reference in their entirety to further clarify the level and scope of the subject matter to which the present disclosure pertains.

As used in the specification and appended claims, the singular forms ("a", "an", and "the") comprise plural referents unless the context clearly states otherwise. Unless the context indicates otherwise, singular terms shall comprise the plural, and plural terms shall comprise the singular. As used in the specification and appended claims, unless stated otherwise, the use of "or" may be used to comprise "and/or".

As used herein, the term "about" may be presented before a particular numerical value. The term "about" used herein comprises not only the exact number recited after the term, but also a range that is near or close to that number. Considering the context in which the number is presented, it may be determined whether any number is close to or near the particular number presented. In one example, the term "about" may refer to a range from -10% to +10% of a numerical value. In another example, the term "about" may refer to a range from -5% to +5% of a given numerical value, but is not limited thereto.

As used herein, the descriptions such as the terms "first, second, third..." "i), ii), iii)..." or "(a), (b), (c), (d)..." are used to distinguish similar constitutions, and these terms do not mean that the constitutions are performed continually or sequentially. For example, when the terms are used in reference to steps of a method, use, or assay, there may be no time interval between these steps, or they may be performed concurrently, or may be performed within several seconds, several minutes, several hours, several days, or several months apart.

As used herein, the term "consisting essentially of" may mean that, in the case where the features of the object claimed herein are not substantially affected by the presence of an unspecified component, the unspecified component may be present.

As used herein, the term, "consisting of" means that the total ratio of specific component(s) is 100%. The components or features that are recited after the term "consisting of" may be essential or mandatory. In some embodiments, in addition to the components or features recited after the term "consisting of", any other components or non-essential components may be excluded.

As used herein, the term "comprising" means the presence of features, steps or components recited after the term, and does not exclude the presence or addition of one or more features, steps or components. The components or features recited after the term "comprising" herein may be essential or mandatory. However, in some embodiments, the term may further comprise any other or non-essential components or features.

As used herein, the term "comprising" may be modified to refer to "consisting essentially of" or "consisting of" in some embodiments.

Regarding amino acid sequences in the present disclosure, although it is described as a polypeptide "comprising" an amino acid sequence described by a specific sequence number, a polypeptide "consisting of" an amino acid sequence described by a specific sequence number, or a protein or polypeptide "having" an amino acid sequence described by a specific sequence number, it is apparent that any protein having an amino acid sequence in which part of the sequence is deleted, modified, substituted, conservatively substituted or added may be used in the present disclosure if it has the same or corresponding activity as the polypeptide consisting of the amino acid sequence of the corresponding sequence number. For example, it may be the case where the N-terminus and/or C-terminus of the amino acid sequence is added with a sequence that does not alter the function of the protein, a naturally occurring mutation, a silent mutation thereof, or a conservative substitution, but is not limited thereto.

As used herein, the term "protein" or "polypeptide" refers to a polymer or oligomer of consecutive amino acid residues. In the present disclosure, "polypeptide," "protein," and "peptide" may be used interchangeably with "amino acid sequence."

In some cases, an amino acid sequence exhibiting activity may be referred to as an "enzyme". In the present disclosure, unless otherwise indicated, amino acid sequences are described in an N-terminal to C-terminal direction.

As used herein, the term "recombinant" with respect to a cell, nucleic acid, polypeptide, or vector indicates that the cell, nucleic acid, polypeptide, or vector has been modified by the introduction of a heterologous nucleic acid or polypeptide or the alteration of a native nucleic acid or polypeptide, or that the cell is derived from a thus modified cell. Therefore, for example, recombinant cells may express genes that are not found within the native (non-recombinant) form of the cells, or may express native genes that are otherwise abnormally expressed, under expressed or not expressed at all.

As used herein, the term "isolated" refers to a substance which exists in a non-naturally occurring environment or does not exist naturally. It comprises at least substantial isolation of a substance (sequence, enzyme, or nucleic acid) from a substance which is naturally associated and found in nature, for example, at least one other component having a sequence, enzyme or nucleic acid.

For example, an isolated sequence, enzyme, or nucleic acid provided herein may be provided in a form that is substantially free of one or more contaminants.

Examples of isolated substances comprise i) any substance that is non-naturally occurring, ii) any substance (e.g., enzyme, variant, nucleic acid, protein, peptide, or cofactor) from which one, or more, or all naturally-occurring components associated in nature have been removed, iii) any substance that has been artificially modified from a substance found in nature, or iv) any substance that has been modified to alter the amount of that substance relative to other components associated in nature (e.g., an increase in the copy number of a gene encoding a specific substance; modification of a promoter naturally linked to a gene encoding a specific substance into a highly active promoter, etc.), but is not limited thereto.

As used herein, the term "wild-type" refers to a naturally-occurring state without artificial modification. When the term "wild-type" is used in reference to a polypeptide, it refers to a naturally occurring polypeptide, that does not have artificial mutations (substitutions, insertions, deletions, etc.) in one or more amino acid positions. Similarly, when the term "wild-type" is used in reference to a polynucleotide, it means not having artificial modifications (substitutions, insertions, deletions) in one or more nucleotides. However, polynucleotides encoding wild-type polypeptides are not limited to wild-type polynucleotides, and comprise sequences encoding any wild-type polypeptides.

As used herein, a parent sequence or backbone refers to a reference sequence in which modifications are introduced to be a variant polypeptide. That is, the parent sequence may serve as a starting sequence to which modifications such as substitutions, additions, and/or deletions, may be introduced. The parent sequence may be naturally-occurring or wild-type, or a variant in which one or more substitutions, insertions or deletions have occurred in the natural or wild type form, or it may be an artificially synthesized sequence. When the parent sequence is an amino acid sequence exhibiting activity, i.e., an amino acid sequence of an enzyme, it may be referred to as a parent enzyme.

As used herein, the term "reference sequence" means a sequence used to determine the position of amino acids within any amino acid sequence. Any amino acid sequence may be aligned with a reference sequence to determine the position of an amino acid that corresponds to a particular position in the reference sequence within any amino acid sequence.

With respect to amino acid or nucleic acid sequences in the present disclosure, the term "fragment" means a part of a parent sequence. For example, it may be a polypeptide in the form in which one or more amino acids of the parent sequence are removed from the C- or N-terminus.

As used herein, the term "fragment" of an enzyme may refer to a "functional fragment". The "functional fragment" may also be referred to as an active fragment, and means a polypeptide that is part of a parent enzyme, and has the enzymatic activity of the parent enzyme. For example, the functional fragment of an enzyme may comprise a catalytic site of the enzyme.

The fragment of the enzyme may comprise a part of the full length of the parent enzyme. For example, the fragment of the enzyme may comprise amino acids of at least about 20%, about 30%, about 40%, about 50%, about 60%, about 70%, about 80%, about 90%, about 95%, about 98%, about 99% or more, or about less than 100% of the full length of the parent enzyme, but is not limited thereto.

As used herein, the term "modifying" means a change or alteration. This may be an alteration from a naturally occurring state. For example, an enzyme may be altered in such a way that the enzyme is altered from a parent sequence or a reference sequence.

In the present disclosure, a modified enzyme may be an enzyme that does not exist as it is in nature, i.e., non-naturally occurring.

As used herein, the term "modified" means alteration, e.g., from its naturally occurring form. The modified enzyme of the present disclosure comprises non-naturally occurring enzymes or naturally occurring variants. For example, the modified enzyme of the present disclosure is a modified enzyme that has not been found in nature. For example, the modified enzyme of the present disclosure may be an enzyme that does not occur spontaneously, but is not limited thereto.

As used herein, the term "modification", when used with respect to amino acid/nucleic acid sequences, may comprise substitution of an amino acid/nucleic acid residue of a parent sequence for a different amino acid/nucleic acid residue in one or more positions in an amino acid sequence, deletion of an amino acid/nucleic acid residue (or series of amino acid/nucleic acid residues) of a parent sequence in one or more positions, insertion of an amino acid/nucleic acid residue (or series of amino acid/nucleic acid residues) of a parent sequence in one or more positions, or truncation of the N-terminal and/or C-terminal amino acid sequences, or 5' and/or 3' nucleic acid sequences, and any combination thereof.

As used herein, the term "variant" or "variant polypeptide (modified polypeptide)" of an enzyme refers to a protein having one or more amino acids different from the parent enzyme by conservative substitution and/or modification. The "variant" or "variant polypeptide" may be used interchangeably. The variant or variant polypeptide may be non-naturally occurring, but is not limited thereto.

The variant differs from the sequence of the parent enzyme by one or more modifications, e.g., substitutions, deletions, and/or insertions of amino acids.

Such variants may generally be identified by modifying one or more amino acids from the parent enzyme and evaluating the properties of the modified protein. In other words, the abilities of the variants may be enhanced, unchanged, or reduced, as compared to the parent enzyme.

Further, some variants may comprise variant polypeptides in which one or more regions, such as an N-terminal leader sequence or transmembrane domain, have been removed.

Other variants may comprise those in which a part has been removed from the N- and/or C-terminal of a mature protein.

The term "variant" or "variant polypeptide" may be used interchangeably with terms such as modification, modified protein, mutant, mutein, divergent, variant, etc., and is not limited as long as the terms are used to indicate mutation.

The variants may also comprise deletion or addition of amino acids that have minimal influence on the properties and secondary structure of a polypeptide. For example, the polypeptide may be conjugated with an N-terminal signal (or leader) sequence of a protein involved in the translocation of proteins co-translationally or post-translationally. Further, the polypeptides may also be conjugated with another sequence or linker to identify, purify, or synthesize the polypeptides.

As used herein, the term "conservative substitution" refers to the substitution of an amino acid with another amino acid having similar structural and/or chemical properties. Such amino acid substitution may generally occur based on the similarity of polarity, charge, solubility, hydrophobicity, hydrophilicity, and/or amphipathic nature of a residue.

Throughout the entire specification of the present disclosure, the conventional one-letter and three-letter codes for naturally occurring amino acids are used. Further, the amino acids mentioned herein are abbreviated according to the nomenclature rules of IUPAC-IUB as follows:

| | |
|---|---|
| Alanine Ala, A | Arginine Arg, R |
| Asparagine Asn, N | Aspartic acid Asp, D |
| Cysteine Cys, C | Glutamic acid Glu, E |
| Glutamine Gln, Q | Glycine Gly, G |
| Histidine His, H | Isoleucine Ile, I |
| Leucine Leu, L | Lysine Lys, K |
| Methionine Met, M | Phenylalanine Phe, F |
| Proline Pro, P | Serine Ser, S |
| Threonine Thr, T | Tryptophan Trp, W |
| Tyrosine Tyr, Y | Valine Val, V |

Meanwhile, any amino acid may be described as Xaa or X.

Further, three-letter codes are generally allowed not only for naturally occurring amino acids, but also for other amino acids, such as 2-aminoisobutyric acid (Aib), Sar (N-methylglycine), α-methyl-glutamic acid, etc.

Amino acids may be generally classified based on the similarity of polarity, charge, solubility, hydrophobicity, hydrophilicity, and/or amphipathic nature of a residue. Accordingly, amino acid substitution may generally occur based on the similarity of polarity, charge, solubility, hydrophobicity, hydrophilicity, and/or amphipathic nature of a residue.

For example, among the amino acids having an electrically charged side chain (electrically charged amino acids), positively charged (basic) amino acids comprise arginine, lysine, and histidine, negatively charged (acidic) amino acids comprise glutamic acid and aspartic acid; among the amino acids having an uncharged side chain (uncharged amino acids), non-polar amino acids comprise glycine, alanine, valine, leucine, isoleucine, methionine, phenylalanine, tryptophan, and proline, polar or hydrophilic amino acids comprise serine, threonine, cysteine, tyrosine, asparagine, and glutamine, and among the non-polar amino acids, aromatic amino acids comprise phenylalanine, tryptophan, and tyrosine.

As used herein, the term "gene" means a polynucleotide that encodes a polypeptide and a polynucleotide comprising regions upstream and downstream of the coding region. In some embodiments, a gene may have a sequence (intron) inserted between each coding region (exon).

As used herein, the term "homology" or "identity" refers to a degree of relevance between two given amino acid sequences or nucleotide sequences, and may be expressed as a percentage. The terms homology and identity may often be used interchangeably with each other.

The sequence homology or identity of conserved polynucleotides or polypeptides may be determined by standard alignment algorithms and may be used with a default gap penalty established by the program being used. Substantially, homologous or identical sequences may generally hybridize under moderately or highly stringent conditions to the full length of the sequence or at least about 50%, about 60%, about 70%, about 80%, or about 90% of the full-length. It is apparent that hybridization also comprises hybridization of a polynucleotide with a polynucleotide comprising a general codon or a codon in consideration of codon degeneracy.

Whether any two polynucleotide or polypeptide sequences have homology, similarity, or identity may be, for example, determined using a known computer algorithm such as the "FASTA" program using default parameters, as in Pearson et al (1988) [Proc. Natl. Acad. Sci. USA 85]: 2444. Alternatively, it may be determined by the Needleman Wunsch algorithm (Needleman and Wunsch, 1970, J. Mol. Biol. 48: 443-453), as performed using the Needleman program of the EMBOSS package (EMBOSS: The European Molecular Biology Open Software Suite, Rice et al., 2000, Trends Genet. 16: 276-277) (version 5.0.0 or later) (GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO et al.](1988) SIAM J Applied Math 48: 1073). For example, homology, similarity, or identity may be determined using BLAST or ClustalW at the National Center for Biotechnology Information, but is not limited thereto.

Homology, similarity, or identity of polynucleotides or polypeptides may be, for example, determined by comparing sequence information using, for example, the GAP computer program, such as Needleman et al. (1970), J Mol Biol. 48:443, as disclosed in Smith and Waterman, Adv. Appl. Math (1981) 2:482. Briefly, the GAP program defines homology, similarity, or identity as the value obtained by dividing the number of similarly aligned symbols (i.e., nucleotides or amino acids) by the total number of the symbols in the shorter of the two sequences. Default parameters for the GAP program may comprise (1) a unitary matrix (comprising a value of 1 for identities and 0 for non-identities) and the weighted comparison matrix of Gribskov et al(1986) Nucl. Acids Res. 14: 6745, as disclosed in Schwartz and Dayhoff, eds., Atlas Of Protein Sequence And Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps.

Further, whether any two polynucleotide or polypeptide sequences have homology, similarity, or identity with each other may be identified by comparing the sequences in a Southern hybridization experiment under stringent conditions as defined, and appropriate hybridization conditions defined are within the skill of the art, and may be determined by a method well known to those skilled in the art (e.g., J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York), but is not limited thereto.

As used herein, the term "mature polypeptide" means a polypeptide in a form without a signal sequence or propeptide sequence. A mature protein/polypeptide/peptide may be a functional form of a protein/polypeptide/peptide. The mature polypeptide may be in a final form after translation or post-translational modification. Examples of the posttranslational modification comprise N- or C-terminal modifications, glycosylation, phosphorylation, leader sequence removal etc., but are not limited thereto.

As used herein, the term "nucleic acid construct" refers to a single- or double-stranded nucleic acid molecule, which comprises one or more regulatory sequences, and which is artificially synthesized, or engineered to comprise a specific sequence in a manner that does not exist in nature, or isolated from nature.

As used herein, the term "expression" comprises any step involved in the production of a polypeptide, e.g., transcription, post-transcriptional modification, translation, post-translational modification, and secretion, etc., but is not limited thereto.

As used herein, the term "expression vector" refers to a linear or circular nucleic acid molecule comprising a coding sequence and an operably linked regulatory sequence for expression thereof.

As used herein, the term "operably linked" refers to a constitution of placing a regulatory sequence to an appropriate position to direct expression of a coding sequence. Thus, the term "operably linked" comprises an attachment or linking between a regulatory region of a functional domain having a known or desired activity such as a promoter, a stop codon, a signal sequence, or an enhancer, and a target (gene or polypeptide) such that the expression, secretion, or function of the target may be regulated in accordance with the known or desired activity.

As used herein, the term "cDNA" refers to a DNA sequence which may be prepared by reverse transcription from a mature, spliced mRNA molecule obtained from a eukaryotic or prokaryotic cell. The cDNA sequence lacks intron sequences that may be present in the corresponding genomic DNA. The initial primary RNA transcript is a precursor to mRNA that is processed through a series of steps comprising splicing before appearing as mature spliced mRNA.

As used herein, the term "regulatory sequence" refers to a polynucleotide sequence necessary for the expression of a coding sequence. Each regulatory sequence may be native (derived from the same origin) or foreign (derived from different genes) to the coding sequence. Examples of the regulatory sequence may comprise a leader sequence, a polyadenylation sequence, a pro-peptide sequence, a promoter, a signal peptide sequence, an operator sequence, a sequence encoding a ribosome-binding domain, and a sequence regulating the termination of transcription and translation. The minimum units of the regulatory sequence may comprise a promoter, and a sequence for terminating transcription and translation.

In order to describe the variants provided in the present disclosure, the following nomenclature is used.

In the present disclosure, referring to a specific position in an amino acid sequence may comprise referring to an amino acid present or substituted at that position. Referring to an amino acid at a specific position may be described in various ways. For example, "position 003" may be described as "position 3", "amino acid 3", or "3rd amino acid". Further, for example, when the amino acid at position 3 is serine (S), it may be described as "S3" or "Ser3".

Amino acid substitution may be expressed by describing the amino acid before substitution, the position, and the amino acid to be substituted in sequence. The amino acids may be expressed using the conventional one-letter and three-letter codes. For example, when alanine, an amino acid at position 6 of a specific sequence, is substituted with valine, it may be described as "A8V" or "Ala8Val".

Any amino acid at a specific position may be referred to as "X". For example, X6 refers to any amino acid at position 6. In addition, when the amino acid to be substituted is expressed as X, it means that it is substituted with an amino acid different from the amino acid present before substitution. For example, "V6X" indicates that V at position 6 is substituted with any amino acid other than V.

Different alterations may be expressed by simultaneously describing several types of amino acids using symbols, such as "/" or ",". For example, when the amino acid (D) at position 12 is substituted with S or K, it may be described in combination of D12S/K or D12S,K. For another example, P/S197K means that the amino acid P or S at position 197 before substitution is substituted with K.

Multiple mutations may be described using "+". For example, descriptions such as "G2A+ M8V" mean that glycine, an amino acid at position 2, is substituted with alanine, and methionine, an amino acid at position 8, is substituted with valine, respectively.

As used herein, the term "corresponding to" refers to an amino acid residue at the position recited in a protein or peptide, or an amino acid residue that is similar, identical, or homologous to the residue recited in a protein or peptide. Identifying an amino acid at a corresponding position may be determining a particular amino acid in a sequence that refers to a particular sequence. As used herein, the "corresponding region" generally refers to a similar or corresponding position in the related protein or reference protein.

In the present disclosure, SEQ ID NO: 1 may be used as a reference sequence to determine the position of amino acids in any amino acid sequence.

In other words, SEQ ID NO: 1 disclosed herein may be used to determine the corresponding amino acid residues in any polypeptide having fumonisin degradation activity. Unless indicated otherwise in the present disclosure, residues of a particular amino acid sequence are numbered, based on SEQ ID NO: 1.

For example, any amino acid sequence is aligned with SEQ ID NO: 1, and based on the alignment, each amino acid residue of the amino acid sequence may be numbered with reference to the numerical position of the amino acid residue corresponding to the amino acid residue of SEQ ID NO: 1. For example, a sequence alignment algorithm such as that described herein may identify the position of an amino acid or a position where modifications such as substitutions, insertions or deletions occur compared to a query sequence (also referred to as a "reference sequence").

In such alignment, the Needleman-Wunsch algorithm (Needleman and Wunsch, 1970, J. Mol. Biol. 48: 443-453), the Needleman program of the EMBOSS package (EMBOSS: The European Molecular Biology Open Software Suite, Rice et al., 2000), Trends Genet. 16: 276-277), etc. may be used, but are not limited thereto.

Additionally, the corresponding amino acid residue in another fumonisin-degrading enzyme may be identified by multiple sequence alignment. Examples of the multiple sequence alignment known in the art comprise programs such as MUSCLE (multiple sequence comparison by log-expectation; version 3.5 or later; Edgar, 2004, Nucleic Acids Research 32: 1792-1797), MAFFT (version 6.857 or later; Katoh and Kuma, 2002, Nucleic Acids Research 30: 3059-3066; Katoh et al., 2005, Nucleic Acids Research 33: 511-518; Katoh and Toh, 2007, Bioinformatics 23: 372-374; Katoh et al., 2009, Methods in Molecular Biology 537: 39-64; Katoh and Toh, 2010, Bioinformatics 26: 1899-1900), and EMBOSS EMMA employing ClustalW (1.83 or later; Thompson et al., 1994, Nucleic Acids Research 22: 4673-4680), and their respective default parameters may be used, but are not limited thereto.

In addition, when enzymes diverged from the mature polypeptide of SEQ ID NO: 1 fail to detect their relationship by traditional sequence-based comparison, other pairwise sequence comparison algorithms may be used (Lindahl and Elofsson, 2000, J. Mol. Biol. 295: 613-615). Greater sensitivity in sequence-based searching may be attained using search programs that utilize probabilistic representations of polypeptide families (profiles) to search databases. For example, the PSI BLAST program generates profiles through an iterative database search process and is capable of detecting remote homologs (Atschul et al., 1997, Nucleic Acids Res. 25: 3389-3402). Even greater sensitivity may be achieved if the family or superfamily of the polypeptide has one or more representatives in the protein structure databases. Programs such as GenTHREADER (Jones, 1999, J. Mol. Biol. 287: 797-815; McGuffin and Jones, 2003, Bioinformatics 19: 874-881) utilize information from a variety of sources, such as PSI BLAST, secondary structure prediction, structural alignment profiles, and solvation potentials, as input to a neural network, which predicts the structural fold for a query sequence. Similarly, the method of Gough et al., 2000, J. Mol. Biol. 313: 903-919 may be used to align a sequence of unknown structure with the superfamily models present in the SCOP database. These alignments may be used in sequence to generate homology models for the peptides, and such models may be assessed for accuracy using a variety of tools developed for that purpose.

For proteins of known structure, several tools and resources are available for retrieving and generating structural alignments. For example, the SCOP superfamilies of proteins have been structurally aligned, and those alignments are accessible and downloadable. Two or more protein structures may be aligned using a variety of algorithms such as the distance alignment matrix (Holm and Sander, 1998, Proteins 33: 88-96) or CE (Combinatiorial extension) Shindyalov and Bourne, 1998, Protein Engineering 11: 739-747). Implementation of these algorithms may additionally be utilized to query structure databases with a structure of interest in order to discover possible structural homologs (Holm and Park, 2000, Bioinformatics 16: 566-567).

The above methods are one exemplary, and are not limited thereto.

Hereinafter, the specific embodiments of the present disclosure will be described in more detail below.

In the present disclosure, the degradation of fumonisin comprises detoxification of fumonisin, inactivation of fumonisin, removal of contamination with fumonisin (decontaminating). Fumonisin may be degraded by some carboxylesterases through a process of producing hydrolyzed FB1 (HFB1) by sequentially generating two tricarboxylic acids (TCAs) through a de-esterification reaction.

In the present disclosure, the fumonisin degradation activity may be measured and assessed using methods known in the art, comprising the embodiments described herein. For example, it may be assessed by measuring the concentration of fumonisin remaining after enzyme treatment.

As used herein, the term "parent fumonisin-degrading enzyme" refers to a fumonisin-degrading enzyme to which a modification is applied to produce the variant or the variant polypeptide of the present disclosure. Specifically, the parent fumonisin-degrading enzyme, parent enzyme, or parent sequence may be a naturally occurring polypeptide or a wild-type polypeptide, may be a mature polypeptide thereof, and may comprise a variant or functional fragment thereof, but is not limited thereto, as long as the polypeptide has fumonisin degradation activity and may be a parent of the variant.

The parent fumonisin-degrading enzyme provided in the present disclosure may be, but is not limited to, a polypeptide of SEQ ID NO: 1. Further, it may be a polypeptide having about 60%, 70%, 75%. 80%. 85%, 90%, 95%, 96%, 97%, 98%, or 99% or more sequence identity to the polypeptide of SEQ ID NO: 1 as long as it has fumonisin degradation activity, and any polypeptide may be comprised within the scope of the parent fumonisin-degrading enzyme as long as it has the activity identical or corresponding to the polypeptide consisting of the amino acid sequence of SEQ ID NO: 1.

The parent fumonisin-degrading enzyme of the variant provided herein may be derived from the *Bradyrhizobium* sp.

Meanwhile, the above-described microorganism is an example of a microorganism from which the parent fumonisin-degrading enzyme provided herein may be derived, and comprises those derived from microorganisms taxonomically homologous to the microorganism regardless of the name of the microorganisms.

The above-described microorganism may be obtained from known microorganism depositary authorities such as ATCC, DSMZ, CBS, NRRL, KCTC, and KCCM.

As used herein, a sequence "derived from" a specific microorganism is not limited to those naturally produced or producible in that microorganism, but also comprises sequences encoded by genes produced by and isolated from the microorganism comprising the genes.

For example, the fumonisin-degrading enzyme derived from *Bradyrhizobium* sp. may comprise not only enzymes having fumonisin degradation activity which are naturally produced by the microorganisms, but also enzymes having carboxylesterase activity derived from the microorganisms, those produced in *Bradyrhizobium* source, and those produced in other host cells through genetic modifications known in the art (e.g., transformation of a sequence encoding the enzyme).

The fumonisin of the present disclosure comprises fumonisin B1, fumonisin B2, fumonisin B3, fumonisin B4, fumonisin A1, and fumonisin A2; and derivatives thereof. For example, the fumonisin of the present disclosure may be selected from fumonisin B1, fumonisin B2, fumonisin B3, fumonisin B4, fumonisin A1, and fumonisin A2. For example, the fumonisin of the present disclosure may be fumonisin B1.

As used herein, the "variant polypeptide having fumonisin degradation activity" may be a variant of the parent fumonisin-degrading enzyme.

As used herein, the "fumonisin-degrading enzyme variant" or "variant polypeptide having fumonisin degradation activity" refers to a protein having fumonisin degradation activity with one or more amino acids that are different from the amino acid sequence of the parent fumonisin-degrading enzyme.

The "variant polypeptide having fumonisin degradation activity", "variant of the parent fumonisin-degrading enzyme", and "fumonisin-degrading enzyme variant" may be used interchangeably.

The variant provided in the present disclosure may comprise one or more amino acid modifications in the sequences of the parent fumonisin-degrading enzyme, while having fumonisin degradation activity. Further, the variant may be i) a polypeptide having 70% or more and less than 100% sequence identity to SEQ ID NO: 1; and/or ii) the variant may be a polypeptide encoded by a polynucleotide having 70% or more and less than 100% sequence identity to a sequence encoding a mature polypeptide of SEQ ID NO: 1; and/or iii) the variant may be a polypeptide encoded by a polynucleotide that hybridizes to (a) a mature polypeptide coding sequence of SEQ ID NO: 1, (b) a cDNA thereof, or (c) the full-length complement of (a) or (b) under low stringency conditions, medium stringency conditions, medium-high stringency conditions, high stringency conditions, or very high stringency conditions; and/or iv) the variant may be a functional fragment of i), ii) or iii) polypeptide having fumonisin degradation activity.

Specifically, the variant provided in the present disclosure comprises modification of one or more amino acids in the parent fumonisin-degrading enzyme sequence while having fumonisin degradation activity, and thus may have one or more modified functions or properties compared to the parent fumonisin-degrading enzyme.

In one embodiment, the variant provided in the present disclosure comprises modification of one or more amino acids in the parent fumonisin-degrading enzyme sequence while having fumonisin degradation activity, and thus may have one or more modified functions or properties compared to the parent fumonisin-degrading enzyme, and one or more conservative substitutions.

The variant provided in the present disclosure, being a variant of the parent fumonisin-degrading enzyme, may be a polypeptide having fumonisin degradation activity.

In one specific embodiment, the variant provided in the present disclosure may comprise modifications at one or more positions of positions 29, 90, 135, 147, 155, 170, 199, 209, 252, 290, 293, 354, 407, and 452 of SEQ ID NO: 1.

In one specific embodiment, the variant provided in the present disclosure may comprise substitutions at one or more positions of positions 29, 90, 135, 147, 155, 170, 199, 209, 252, 290, 293, 354, 407, and 452 of SEQ ID NO: 1.

In the present disclosure, the position number is a position corresponding to the position of the polypeptide of SEQ ID NO: 1, and the term "corresponding" is as described above.

In one specific embodiment, the variant provided in the present disclosure may comprise modifications of amino acids corresponding to one or more of G29, N90, A135, A147, A155, L170, V199, V209, T252, T290, I293, R354, A407, and M452 of SEQ ID NO: 1.

In one specific embodiment, in SEQ ID NO: 1 before the modification provided in the present disclosure, the amino acid corresponding to position 29 may be G; the amino acid corresponding to position 90 may be N; the amino acid corresponding to position 135 may be A; the amino acid corresponding to position 147 may be A; the amino acid corresponding to position 155 may be A; the amino acid corresponding to position 170 may be L; the amino acid corresponding to position 199 may be V; the amino acid corresponding to position 209 may be V; the amino acid corresponding to position 252 may be T; the amino acid corresponding to position 290 may be T; the amino acid corresponding to position 293 may be I; the amino acid corresponding to position 354 may be R; the amino acid corresponding to position 407 may be A; and/or the amino acid corresponding to position 452 may be M.

In one specific embodiment, the variant provided in the present disclosure may comprise a substitution of the amino acid corresponding to position 29 of SEQ ID NO: 1 with A, V, L, I, M, F, W, P, S, T, C, Y, N, Q, D, E, K, R, or H, and specifically, a substitution with alanine (A).

In one specific embodiment, the variant provided in the present disclosure may comprise a substitution of the amino acid corresponding to position 90 of SEQ ID NO: 1 with G, A, V, L, I, M, F, W, P, S, T, C, Y, Q, D, E, K, R, or H, and specifically, a substitution with valine (V) or alanine (A).

In one specific embodiment, the variant provided in the present disclosure may comprise a substitution of the amino acid corresponding to position 135 of SEQ ID NO: 1 with G, V, L, I, M, F, W, P, S, T, C, Y, N, Q, D, E, K, R, or H, and specifically, a substitution with leucine (L).

In one specific embodiment, the variant provided in the present disclosure may comprise a substitution of the amino acid corresponding to position 147 of SEQ ID NO: 1 with G, V, L, I, M, F, W, P, S, T, C, Y, N, Q, D, E, K, R, or H, specifically, a substitution with S, T, C, Y, N, or Q, and more specifically, a substitution with serine (S) or threonine (T).

In one specific embodiment, the variant provided in the present disclosure may comprise a substitution of the amino acid corresponding to position 155 of SEQ ID NO: 1 with G, V, L, I, M, F, W, P, S, T, C, Y, N, Q, D, E, K, R, or H, and specifically, a substitution with serine (S).

In one specific embodiment, the variant provided in the present disclosure may comprise a substitution of the amino acid corresponding to position 170 of SEQ ID NO: 1 with G, A, V, I, M, F, W, P, S, T, C, Y, N, Q, D, E, K, R, or H, and specifically, a substitution with valine (V).

In one specific embodiment, the variant provided in the present disclosure may comprise a substitution of the amino acid corresponding to position 199 of SEQ ID NO: 1 with G, A, L, I, M, F, W, P, S, T, C, Y, N, Q, D, E, K, R, or H, and specifically, a substitution with leucine (L).

In one specific embodiment, the variant provided in the present disclosure may comprise a substitution of the amino acid corresponding to position 209 of SEQ ID NO: 1 with G, A, L, I, M, F, W, P, S, T, C, Y, N, Q, D, E, K, R, or H, and specifically, a substitution with phenylalanine (F).

In one specific embodiment, the variant provided in the present disclosure may comprise a substitution of the amino acid corresponding to position 252 of SEQ ID NO: 1 with G, A, V, L, I, M, F, W, P, S, C, Y, N, Q, D, E, K, R, or H, and specifically, a substitution with leucine (L).

In one specific embodiment, the variant provided in the present disclosure may comprise a substitution of the amino acid corresponding to position 290 of SEQ ID NO: 1 with G, A, V, L, I, M, F, W, P, S, C, Y, N, Q, D, E, K, R, or H, and specifically, a substitution with alanine (A).

In one specific embodiment, the variant provided in the present disclosure may comprise a substitution of the amino acid corresponding to position 293 of SEQ ID NO: 1 with G, A, V, L, M, F, W, P, S, T, C, Y, N, Q, D, E, K, R, or H, and specifically, a substitution with glutamine (Q).

In one specific embodiment, the variant provided in the present disclosure may comprise a substitution of the amino acid corresponding to position 354 of SEQ ID NO: 1 with G, A, V, L, I, M, F, W, P, S, T, C, Y, N, Q, D, E, K, or H, and specifically, a substitution with alanine (A).

In one specific embodiment, the variant provided in the present disclosure may comprise a substitution of the amino acid corresponding to position 407 of SEQ ID NO: 1 with G, V, L, I, M, F, W, P, S, T, C, Y, N, Q, D, E, K, R, or H, and specifically, a substitution with glycine (G).

In one specific embodiment, the variant provided in the present disclosure may comprise a substitution of the amino acid corresponding to position 452 of SEQ ID NO: 1 with G, A, V, L, I, F, W, P, S, T, C, Y, N, Q, D, E, K, R, or H, and specifically, a substitution with glycine (G).

In one specific embodiment, the variant provided in the present disclosure may comprise one or more substitutions of A147S/T, A155S, N90V, A135L, L170V, V209F, T252L, I293Q, R354A, A407G, V199L, G29A, M452G, and T290A in SEQ ID NO: 1.

Specifically, the variant comprising the substitutions of A147S+A155S in SEQ ID NO: 1 may be represented by SEQ ID NO: 9, the variant comprising the substitution of N90V in SEQ ID NO: 1 may be represented by SEQ ID NO: 10, the variant comprising the substitution of A135L in SEQ ID NO: 1 may be represented by SEQ ID NO: 3, the variant comprising the substitution of A155S in SEQ ID NO: 1 may be represented by SEQ ID NO: 11, the variant comprising the substitution of L170V in SEQ ID NO: 1 may be represented by SEQ ID NO: 12, the variant comprising the substitution of V209F in SEQ ID NO: 1 may be represented by SEQ ID NO: 13, the variant comprising the substitution of T252L in SEQ ID NO: 1 may be represented by SEQ ID NO: 14, the variant comprising the substitutions of A135L+N90V in SEQ ID NO: 1 may be represented by SEQ ID NO: 29, the variant comprising the substitutions of A135L+A147S+A155S in SEQ ID NO: 1 may be represented by SEQ ID NO: 30, the variant comprising the substitutions of A135L+A155S in SEQ ID NO: 1 may be represented by SEQ ID NO: 31, the variant comprising the substitutions of A135L+L170V in SEQ ID NO: 1 may be represented by SEQ ID NO: 32, the variant comprising the substitutions of A135L+I293Q in SEQ ID NO: 1 may be represented by SEQ ID NO: 33, the variant comprising the substitutions of A135L+N90A+R354A in SEQ ID NO: 1 may be represented by SEQ ID NO: 36, the variant comprising the substitutions of A135L+A147T in SEQ ID NO: 1 may be represented by SEQ ID NO: 37, the variant comprising the substitutions of A135L+A407G in SEQ ID NO: 1 may be represented by SEQ ID NO: 5, the variant comprising the substitutions of A135L+V199L in SEQ ID NO: 1 may be represented by SEQ ID NO: 38, the variant comprising the substitutions of A135L+A407G+G29A in SEQ ID NO: 1 may be represented by SEQ ID NO: 7, the variant comprising the substitutions of A135L+A407G+M452G in SEQ ID NO: 1 may be represented by SEQ ID NO: 49, the variant comprising the substitutions of A135L+A407G+A147T in SEQ ID NO: 1 may be represented by SEQ ID NO: 50, the variant comprising the substitutions of A135L+A407G+T290A in SEQ ID NO: 1 may be represented by SEQ ID NO: 51, the variant comprising the substitutions of A135L+A407G+V199L in SEQ ID NO: 1 may be represented by SEQ ID NO: 52, the variant comprising the substitutions of A135L+A407G+G29A+M452G in SEQ ID NO: 1 may be represented by SEQ ID NO: 59, and the variant comprising the substitutions of A135L+A407G+G29A+V199L in SEQ ID NO: 1 may be represented by SEQ ID NO: 60.

In one specific embodiment, the variant provided in the present disclosure comprises all possible combinations of the modifications described above.

In one specific embodiment, the variant may have a substitution of any one of the amino acids at positions 90, 135, 155, 170, 209, and 225 with a different amino acid.

In one specific embodiment, the variant may have substitutions of any one or more of the amino acids at positions 293, 407, 29, 452, and 290 with different amino acids.

For example, the variant may comprise modifications of amino acids at positions selected below, through combinations of the modifications described above:
147+155;
90;
135;
155;
170;
209;
252;
135+90;
135+147+155;
135+155;
135+170;
135+293;
135+90+354;
135+147;
135+407;
135+199;
135+407+29;
135+407+452;
135+407+147;
135+407+290;
135+407+199;
135+407+29+452; and
135+407+29+199.

In another example, the variant may comprise, but is not limited to, any one or more substitutions of the following i) to xiv):
i) a substitution of the amino acid at position 29 with alanine (A);
ii) a substitution of the amino acid at position 90 with valine (V) or alanine (A);
iii) a substitution of the amino acid at position 135 with leucine (L);
iv) a substitution of the amino acid at position 147 with serine (S) or threonine (T);
v) a substitution of the amino acid at position 155 with serine (S);
vi) a substitution of the amino acid at position 170 with valine (V);
vii) a substitution of the amino acid at position 199 with leucine (L);
viii) a substitution of the amino acid at position 209 with phenylalanine (F);
ix) a substitution of the amino acid at position 252 with leucine (L);
x) a substitution of the amino acid at position 290 with alanine (A);
xi) a substitution of the amino acid at position 293 with glutamine (Q);
xii) a substitution of the amino acid at position 354 with alanine (A);
xiii) a substitution of the amino acid at position 407 with glycine (G); and
xiv) a substitution of the amino acid at position 452 with glycine(G).

For still another example, the variant may comprise, but is not limited to, any one or more modifications selected from the following modifications:
A147S+A155S;
N90V;
A135L;
A155S;
L170V;
V209F;
T252L;
A135L+N90V;
A135L+A147S+A155S;
A135L+A155S;
A135L+L170V;
A135L+I293Q;
A135L+N90A+R354A;
A135L+A147T;
A135L+A407G;
A135L+V199L;
A135L+A407G+G29A;
A135L+A407G+M452G;
A135L+A407G+A147T;
A135L+A407G+T29A;
A135L+A407G+V199L; and
A135L+A407G+G29A+V199L.

In one specific embodiment, the variant provided in the present disclosure may have about 60% or more, for example, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more and less than 100% sequence identify to the parent fumonisin- degrading enzyme; a mature polypeptide thereof, or a functional fragment thereof.

In one specific embodiment, the variant provided in the present disclosure may have about 60% or more, for example, 65% or more, 70% or more, 75% or more, 80% or more, 85% or more, 90% or more, 91% or more, 92% or more, 93% or more, 94% or more, 95% or more, 96% or more, 97% or more, 985 or more, or 99% or more and aless than 100% sequence identify to SEQ ID NO: 1.

In one specific embodiment, the variant provided in the present disclosure may have about 60% or more, for example 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more and less than 100% sequence identity to a sequence encoding a mature polypeptide of SEQ ID NO: 1.

In on specific embodiment, the variant provided in the present disclosure may have about 60% or more, for example, 65% or more, 70% or more, 75% or more, 80% or more, 85% or more, 90% or more, 91% or more, 92% or more, 93% or more, 94% or more, 95% or more, 96% or more, 97% or more, 98% or more, or 99% or more and less than 100% sequence identity to a fucntional fragment of SEQ ID NO: 1.

In the variant provided in the present disclosure, one or more of any selectable or detectable properties or attributes of a polypeptide may be altered as compared to other fumonisin-degrading enzymes, e.g., a wild-type fumonisin- degrading enzyme, parent fumonisin-degrading enzyme, other fumonisin- degrading enzyme variants, etc.

The properties or attributes may comprise, but are not limited to, oxidative stability, substrate specificity, catalytic activity, thermal stability, alkaline stability, pH activity profile, resistance to proteolytic degradation, Km, kcat, kcat/Km ratio, protein folding, inducing an immune response, ability to bind to a ligand, ability to bind to a receptor, ability to be secreted, ability to be displayed on the surface of a cell, ability to oligomerize, ability to signal, ability to stimulate cell proliferation, ability to inhibit cell proliferation, ability to induce apoptosis, ability to be modified by phosphorylation or glycosylation, and/or ability to treat diseases, etc.

Specifically, the variant provided in the present disclosure may have any one or more of the following modified activities compared to the parent sequence:
i) increased or decreased enzymatic activity; and
ii) increased or decreased specific activity.

More specifically, the variant provided in the present disclosure may have increased enzymatic activity; and/or increased specific activity, as compared to the parent sequence, but is not limited thereto.

As used herein, the "enzymatic activity" represents at least one catalytic activity. Specifically, it may be the conversion efficiency of an enzyme mainly expressed as kcat/Km, but is not limited thereto.

When the enzyme is completely saturated with substrates, kcat means the rate constant (catalytic constant) that converts substrates into products in unit time by one enzyme, and is also referred to as a turnover number. Km is the substrate concentration when the reaction rate is at half the maximum value (Vmax).

Examples of the ways to express enzymatic activity comprise specific activity (umol of converted substrate x mg⁻¹ x min⁻¹) or volumetric activity (umol of converted substrate x mL⁻¹ x min⁻¹), etc.

However, defining the enzymatic activity is not limited to the description described above, and the enzymatic activity may be defined and evaluated based on the information disclosed in Irwin H. Segel, Enzyme kinetics, John Wiley & Sons, 1979; A. G. Marangoni, Enzyme kinetics, Wiley-Interscience, 2003; A. Fersht, Enzyme structure and mechanisms, John Wiley & Sons, 1981; Structure and Mechanism in Protein Science: A guide to enzyme catalysis and protein folding, Alan Fersht, W.H. Freeman, 1999; Fundamentals of Enzyme Kinetics, Athel Cornish-Bowden, Wiley-Blackwell 2012 and Voet ef al., "Biochemie" [Biochemistry], 1992, VCH-Verlag, Chapter 13, pages 331-332 with respect to enzymatic activity, etc. For another example, the enzymatic activity may be evaluated by measuring the concentration of the substrate fumonisin.

In one specific embodiment, the variant provided in the present disclosure may have an increased enzymatic activity of about 100%, about 110%, about 120%, about 130%, about 140%, about 150%, about 160%, about 170%, about 180%, about 190%, or about 200% or more compared to the parent enzyme.

As used herein, the term "specific activity" is the activity of an enzyme per unit weight of proteins, and may be expressed as unit/mg. The quantification of proteins may be performed using, for example, SDS-PAGE or Bradford assay.

The polynucleotide encoding the variant of the present disclosure may comprise the coding sequence of the above-described variant. The polynucleotide may undergo various modifications in the coding region within the scope that does not change the amino acid sequence of the polypeptide, due to codon degeneracy or in consideration of the codons preferred in an organism in which the polypeptide is to be expressed.

Further, the polynucleotide of the present disclosure may comprise a probe that may be prepared from a known gene sequence, for example, any sequence encoding the variant of the present disclosure by hybridizing with a sequence complementary to all or part of the nucleotide sequence described above under stringent conditions without limitation.

The term "stringent conditions" refers to conditions under which specific hybridization between polynucleotides is allowed. Such conditions are specifically described in a literature (e.g., J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York).

For example, the stringent conditions may comprise conditions under which polynucleotides having a high homology or identity of 40% or more, specifically, 90% or more, more specifically 95% or more, 96% or more, 97% or more, 98% or more, more specifically, 99% or more are hybridized with each other, and polynucleotides having a homology or identity lower than the above are not hybridized with each other, or washing conditions of the conventional Southern hybridization, that is, washing once, specifically, twice or three times at a salt concentration and a temperature corresponding to 60°C, 1XSSC, 0.1% SDS, specifically, 60°C, 0.1XSSC, 0.1% SDS, and more specifically, 68°C, 0.1XSSC, 0.1% SDS.

Hybridization requires that two nucleic acids comprise complementary sequences, although mismatches between bases are possible depending on the stringency of the hybridization. The term "complementary" is used to describe the relationship between nucleotide bases that are able to hybridize with each other. For example, with respect to DNA, adenine is complementary to thymine, and cytosine is complementary to guanine. Therefore, the polynucleotide of the present disclosure may comprise isolated nucleotide fragments complementary to the entire sequence as well as nucleic acid sequences substantially similar thereto.

Specifically, polynucleotides having homology or identity may be detected using the hybridization conditions comprising a hybridization step at a Tm value of 55°C under the above described conditions. Further, the Tm value may be 60°C, 63°C, or 65°C, but is not limited thereto, and may be appropriately adjusted by those skilled in the art depending on the purpose thereof.

The appropriate stringency for hybridizing the polynucleotides depends on the length of the polynucleotides and the degree of complementation, and these variables are well known in the art (see, Sambrook et al., supra, 9.50-9.51, 11.7-11.8).

For example, the "high stringency" may occur at about 5°C to about 10°C below the Tm of the probe; the "medium stringency" may occur at about 10°C to about 20°C below the Tm of the probe; and the "low stringency" may occur at about 20°C to about 25°C below the Tm, but the stringency is not limited thereto.

For example, the "low stringency condition" may mean, for probes of at least about 100 nucleotides in length, prehybridization and hybridization at 42°C in 5X SSPE, 0.3% SDS, 200 micrograms/ml of sheared and denatured salmon sperm DNA, and 25% formamide, according to the standard Southern blotting procedures for 12 hours to 24 hours. The carrier material may be finally washed two or three times each for 15 minutes in 2 X SSC, 0.1% to 0.2% SDS at 50°C.

For example, the "medium stringency condition" may mean, for probes of at least about 100 nucleotides in length, prehybridization and hybridization at 42°C in 5X SSPE, 0.3% SDS, 200 micrograms/ml of sheared and denatured salmon sperm DNA, and 35% formamide, according to the standard Southern blotting procedures for 12 hours to 24 hours. The carrier material may be finally washed two or three times each for 15 minutes in 2 X SSC, 0.1% to 0.2% SDS at 55°C. For example, the "medium-high stringency condition" may mean, for probes of at least about 100 nucleotides in length, prehybridization and hybridization at 42°C in 5X SSPE, 0.3% SDS, 200 micrograms/ml of sheared and denatured salmon sperm DNA, and 35% formamide, according to the standard Southern blotting procedures for 12 hours to 24 hours. The carrier material may be finally washed two or three times each for 15 minutes in 1 to 2 X SSC, 0.1% to 0.2% SDS at 60°C.

For example, the "high stringency condition" may mean, for probes of at least about 100 nucleotides in length, prehybridization and hybridization at 42°C in 5X SSPE, 0.3% SDS, 200 micrograms/ml of sheared and denatured salmon sperm DNA, and 35% formamide, according to the standard Southern blotting procedures for 12 hours to 24 hours. The carrier material is finally washed two or three times each for 15 minutes in 2 X SSC, 0.1% to 0.2% SDS at 65°C.

The nucleic acid construct provided in the present disclosure may comprise a polynucleotide encoding the variant provided herein, operably linked to one or more regulatory sequences that direct the expression of the coding sequence in an appropriate host cell under conditions suitable for the regulatory sequences.

The polynucleotides may be engineered in various ways to allow the expression of variants. Depending on the expression vector, it may be desirable or necessary to manipulate the polynucleotides prior to insertion into the vector. Such manipulations may be performed using methods known in the art.

The "vector" provided in the present disclosure refers to a DNA construct comprising the nucleotide sequence of a polynucleotide encoding the variant operably linked to a suitable expression regulatory region (expression regulatory sequence) so as to be able to express the variant of the present disclosure in a suitable host cell. The expression regulatory region may comprise a promoter capable of initiating transcription, any operator sequence for regulating such transcription, a sequence encoding a suitable mRNA ribosome-binding site, and a sequence for regulating termination of transcription and translation. Once transformed into a suitable host cell, the vector may replicate or function independently from the host genome, or may integrate into genome thereof.

The vector that may be used in the present disclosure is not particularly limited, and any vector known in the art may be used. Examples of the vectors commonly used may comprise natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A, etc. may be used; and as a plasmid vector, those based on pBR, pUC, pBluescriptll, pGEM, pTZ, pCL and pET, etc. may be used. Specifically, pDZ, pACYC177, pACYC184, pCL, pECCG117, pUC19, pBR322, pMW118, pCC1BAC vector, etc. may be used.

For example, a polynucleotide encoding the variant provided in the present disclosure may be inserted into the chromosome through a vector for intracellular chromosomal insertion. The insertion of the polynucleotide into the chromosome may be performed by any method known in the art, for example, by homologous recombination, but is not limited thereto. The vector may further comprise a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cytotoxic agents, or expression of surface polypeptides, may be used. Only cells expressing the selection marker are able to survive or show different phenotypes under the environment treated with the selective agent, and thus the transformed cells may be selected.

Any host cell may be comprised without limitation in the host cell of the present disclosure as long as it is able to express the variant of the present disclosure.

The host cell of the present disclosure may comprise the above-described variant, a polynucleotide encoding the variant, a nucleic acid construct and/or vector comprising the same.

The nucleic acid construct or vector may be integrated into the chromosome as described earlier, or may remain as a self-replicating extrachromosomal vector.

The host cell of the present disclosure encompasses any progeny of a parent cell that is not identical to the parent cell due to mutations that occur during replication.

The host cell may be any cell useful in the recombinant production of a variant, e.g., a prokaryotic cell or a eukaryotic cell.

The prokaryotic host cell may be any Gram-positive or Gram-negative bacterium.

The Gram-positive bacteria comprise, but are not limited to, *Bacillus*, *Clostridium*, *Enterococcus*, *Geobacillus*, *Lactobacillus*, *Lactococcus*, *Oceanobacillus*, *Staphylococcus*, *Streptococcus*, and *Streptomyces*.

The Gram-negative bacteria comprise, but are not limited to, *Campylobacter*, *Escherichia coli*, *Flavobacterium*, *Fusobacterium*, *Helicobacter*, *Ilyobacter*, *Neisseria*, *Pseudomonas*, *Salmonella*, *Vibrio* (e.g., *Vibrio natriegens*), and *Ureaplasma*.

In one specific embodiment, the bacterial host cell may be a host cell belonging to the genus *Bacillus*, specifically, comprising *Bacillus alkalophilus*, *Bacillus amyloliquefaciens*, *Bacillus brevis*, *Bacillus circulans*, *Bacillus clausii*, *Bacillus coagulans*, *Bacillus firmus*, *Bacillus lautus*, *Bacillus lentus*, *Bacillus licheniformis*, *Bacillus megaterium*, *Bacillus pumilus*, *Bacillus stearothermophilus*, *Bacillus subtilis*, and *Bacillus thuringiensis* cells, but is not limited thereto.

In one specific embodiment, the bacterial host cell may be a host cell belonging to the genus *Streptococcus*, specifically, *Streptococcus equisimilis*, *Streptococcus pyogenes*, *Streptococcus uberis*, and *Streptococcus equi subsp. Zooepidemicus* cells, but is not limited thereto.

In one specific embodiment, the bacterial host cell may be a host cell belonging to the genus *Streptomyces*, specifically, *Streptomyces achromogenes*, *Streptomyces avermitilis*, *Streptomyces coelicolor*, *Streptomyces griseus*, and *Streptomyces lividans* cells, but is not limited thereto.

In one specific embodiment, the bacterial host cell may be a host cell belonging to the genus *Corynebacterium*, specifically, *Corynebacterium glutamicum*, *Corynebacterium crudilactis*, *Corynebacterium deserti*, *Corynebacterium efficiens*, *Corynebacterium callunae*, *Corynebacterium stationis*, *Corynebacterium singulare*, *Corynebacterium halotolerans*, *Corynebacterium striatum*, *Corynebacterium ammoniagenes*, *Corynebacterium pollutisoli*, *Corynebacterium imitans*, *Corynebacterium testudinoris*, or *Corynebacterium flavescens*, but is not limited thereto.

The host cell may be a eukaryotic cell, such as a mammalian, insect, plant, or fungal cell.

The host cell may be a fungal cell. As used herein, the "fungi" may comprise *Ascomycota*, *Basidiomycota*, *Chytridiomycota*, and *Zygomycota* as well as the *Oomycota* and all mitosporic fungi.

The fungal host cell may be a yeast cell. As used herein, the "yeast" comprises *ascosporogenous* yeast (*Endomycetales*), *basidiosporogenous* yeast, and yeast belonging to the Fungi imperfecti (*Blastomycetes*). However, this classification may change, and may be defined as described in Biology and Activities of Yeast (Skinner, Passmore, and Davenport, editors, Soc. App. Bacteriol. Symposium Series No. 9, 1980).

The yeast host cell may be a *Candida*, *Hansenula*, *Kluyveromyces*, *Pichia*, *Komagataella*, *Saccharomyces*, *Schizosaccharomyces*, or *Yarrowia* cell, for example, *Kluyveromyces lactis*, *Saccharomyces carlsbergensis*, *Saccharomyces cerevisiae*, *Saccharomyces diastaticus*, *Saccharomyces douglasii*, *Saccharomyces kluyveri*, *Saccharomyces norbensis*, *Saccharomyces oviformis*, *Komagataella phaffii*, or *Yarrowia lipolytica* cell.

The fungal host cell may be a filamentous fungal cell. As used herein, the "filamentous fungi" comprise all filamentous forms of the subdivision *Eumycota* and *Oomycota* (as defined by the above literature (Hawksworth et al., 1995)). The filamentous fungi are generally characterized by a mycelial wall composed of chitin, cellulose, glucan, chitosan, mannan, and other complex polysaccharides. Vegetative growth is by hyphal elongation, and carbon catabolism is obligately aerobic. In contrast, vegetative growth by yeasts such as *Saccharomyces cerevisiae* is by budding of a unicellular thallus, and carbon catabolism may be fermentative.

The filamentous fungal host cell may be an *Acremonium*, *Aspergillus*, *Aureobasidium*, *Bjerkandera*, *Ceriporiopsis*, *Chrysosporium*, *Coprinus*, *Coriolus*, *Cryptococcus*, *Filibasidium*, *Fusarium*, *Humicola*, *Magnaporthe*, *Mucor*, *Myceliophthora*, *Neocallimastix*, *Neurospora*, *Paecilomyces*, *Penicillium*, *Phanerochaete*, *Phlebia*, *Piromyces*, *Pleurotus*, *Schizophyllum*, *Talaromyces*, *Thermoascus*, *Thielavia*, *Tolypocladium*, *Trametes*, or *Trichoderma* cell.

For example, the filamentous fungal host cell may be an *Aspergillus awamori*, *Aspergillus foetidus*, *Aspergillus fumigatus*, *Aspergillus japonicus*, *Aspergillus nidulans*, *Aspergillus niger*, *Aspergillus oryzae*, *Bjerkandera adusta*, *Ceriporiopsis aneirina*, *Ceriporiopsis caregiea*, *Ceriporiopsis gilvescens*, *Ceriporiopsis pannocinta*, *Ceriporiopsis rivulosa*, *Ceriporiopsis subrufa*, *Ceriporiopsis subvermispora*, *Chrysosporium inops*, *Chrysosporium keratinophilum*, *Chrysosporium lucknowense*, *Chrysosporium merdarium*, *Chrysosporium pannicola*, *Chrysosporium queenslandicum*, *Chrysosporium tropicum*, *Chrysosporium zonatum*, *Coprinus cinereus*, *Coriolus hirsutus*, *Fusarium bactridioides*, *Fusarium cerealis*, *Fusarium crookwellense*, *Fusarium culmorum*, *Fusarium graminearum*, *Fusarium graminum*, *Fusarium heterosporum*, *Fusarium negundi*, *Fusarium oxysporum*, *Fusarium reticulatum*, *Fusarium roseum*, *Fusarium sambucinum*, *Fusarium sarcochroum*, *Fusarium sporotrichioides*, *Fusarium sulphureum*, *Fusarium torulosum*, *Fusarium trichothecioides*, *Fusarium venenatum*, *Humicola insolens*, *Humicola lanuginosa*, *Mucor miehei*, *Myceliophthora thermophila*, *Neurospora crassa*, *Penicillium purpurogenum*, *Phanerochaete chrysosporium*, *Phlebia radiata*, *Pleurotus eryngii*, *Thielavia terrestris*, *Trametes villosa*, *Trametes versicolor*, *Trichoderma harzianum*, *Trichoderma koningii*, *Trichoderma longibrachiatum*, *Trichoderma reesei*, or *Trichoderma viride* cell. However, the filamentous fungal host cell is not limited thereto.

The composition of the present disclosure may be used for degrading fumonisin.

In one embodiment, the fumonisin may be present in foods.

In one embodiment, the fumonisin may be present in feeds.

The composition of the present disclosure may be used to degrade and detoxify fumonisin present in foods and/or feeds.

The composition of the present disclosure may further comprise other components, in addition to the variant provided in the present disclosure. The components added to the composition of the present disclosure may be appropriately selected by those skilled in the art.

In one specific embodiment, the composition of the present disclosure may further comprise any components suitable for being applied to fumonisin degradation.

In one specific embodiment, the composition of the present disclosure may further comprise any components suitable for being applied to food and/or feed additives.

Examples of materials that may be added comprise stabilizers, surfactants, builders, chelating agents, dispersants, enzymes, enzyme stabilizers, catalysts, activators, carriers, binders, lubricants, disintegrants, excipients, solubilizers, suspending agents, colorants, flavoring agents, buffers, preservatives, analgesics, solubilizers, isotonic agents, stabilizers, diluents, lubricants, preservatives, etc., but are not limited thereto.

In one specific embodiment, the composition provided in the present disclosure may further comprise a naturally occurring material or a non-naturally occurring material, in addition to the variant provided in the present disclosure.

In one specific embodiment, the composition provided in the present disclosure may further comprise additional enzymes used in food and/or feed additives, in addition to the variant provided in the present disclosure.

The method of preparing the variant of the present disclosure may comprise the step of culturing a host cell; and the step of recovering the variant expressed in the culturing step.

As used herein, the term "culturing" means that the host cell is grown under appropriately controlled environmental conditions. The culturing process of the present disclosure may be performed in a suitable medium and culture conditions known in the art. Such a culturing process may be easily adjusted for use by those skilled in the art according to the strain to be selected. Specifically, the culture may be a batch culture, a continuous culture, and a fed-batch culture, but is not limited thereto.

As used herein, the term "medium" refers to a mixture of materials which comprise, as a main ingredient, nutrient materials required for culturing the host cell, and it supplies nutrient materials and growth factors, along with water that is essential for survival and growth. Specifically, the medium and other culture conditions used for culturing the host cell of the present disclosure may be any medium commonly used for culturing host cells without any particular limitation. However, the host cell of the present disclosure may be cultured under aerobic conditions in a common medium comprising appropriate carbon sources, nitrogen sources, phosphorus sources, inorganic compounds, amino acids, and/or vitamins, etc., while adjusting temperature, pH, etc.

In the present disclosure, the carbon sources may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; amino acids, such as glutamic acid, methionine, lysine, etc. Further, the carbon sources may comprise natural organic nutrients such as starch hydrolysate, molasses, blackstrap molasses, rice bran, cassava, sugar cane molasses, and corn steep liquor, etc. Specifically, carbohydrates such as glucose and sterilized pretreated molasses (i.e., molasses converted to reducing sugar) may be used, and in addition, various other carbon sources may be used in an appropriate amount without limitation. These carbon sources may be used alone or in combination of two or more thereof, but are not limited thereto.

The nitrogen sources may comprise inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extract, yeast extract, malt extract, corn steep liquor, casein hydrolysate, fish or decomposition product thereof, defatted soybean cake or decomposition product thereof, etc. These nitrogen sources may be used alone or in combination of two or more thereof, but are not limited thereto.

The phosphorus sources may comprise monopotassium phosphate, dipotassium phosphate, or corresponding sodium-comprising salts, etc. The inorganic compounds may comprise sodium chloride, calcium chloride, iron chloride, magnesium sulfate, iron sulfate, manganese sulfate, calcium carbonate, etc. In addition, amino acids, vitamins, and/or appropriate precursors, etc. may be comprised. These components or precursors may be added to a medium in a batch or continuous manner, but these phosphorus sources are not limited thereto.

Further, pH of the medium may be adjusted by adding a compound such as ammonium hydroxide, potassium hydroxide, ammonia, phosphoric acid, sulfuric acid, etc. during the culturing of the host cell in an appropriate manner. In addition, bubble formation may be prevented during culturing by using an antifoaming agent such as fatty acid polyglycol ester. Further, oxygen gas or a gas comprising oxygen may be injected into the medium to maintain aerobic conditions of the medium, or nitrogen gas, hydrogen gas, or carbon dioxide may be injected to maintain anaerobic or microaerobic conditions, without the injection of gas, but are not limited thereto.

The temperature of the medium may be 20°C to 40°C, specifically, 25°C to 37°C, but is not limited thereto. The culturing may be continued until the desired amount of a useful material is obtained, and may be specifically carried out for 10 hours to 120 hours, but is not limited thereto.

In one specific embodiment, the variant expressed during the culturing step may be recovered using methods known in the art to which the present disclosure pertains. For example, the variant may be recovered from a nutrient medium by common procedures comprising, but are not limited to, collection, centrifugation, filtration, extraction, spray-drying, evaporation, or precipitation.

The recovery method is to collect the variant using the method of culturing the host cell of the present disclosure, for example, using a suitable method known in the art according to a batch culture, continuous culture, or fed-batch culture method. For example, methods such as centrifugation, filtration, treatment with a protein crystallizing precipitant (salting-out method), extraction, ultrasonic disruption, ultrafiltration, dialysis, various kinds of chromatography such as molecular sieve chromatography (gel filtration), adsorption chromatography, ion exchange chromatography, affinity chromatography, etc., HPLC, and a combination thereof may be used, and the variant may be recovered from the medium or the host cell using suitable methods known in the art.

In another specific embodiment, the variant expressed by the host cell in the culturing step may not be recovered. In the specific embodiment, the host cell expressing the variant as it is may be used as a source of the variant.

### [Mode for Carrying Out the Invention]

Hereinafter, the present disclosure will be described in more detail with reference to Examples and Experimental Examples. However, these Examples and Experimental Examples are only for illustrating the present disclosure, and the scope of the present disclosure is not intended to be limited by these Examples and Experimental Examples.

### Example 1: Preparation of CB1 variants

### Example 1-1. Preparation of CB1

A polynucleotide encoding one kind of variant (hereinafter referred to as CB1, SEQ ID NO: 1) comprising multiple mutations in a carboxylesterase family protein sequence derived from the *Bradyrhizobium* sp. was synthesized by Cosmo Genetech and cloned into a pET vector (Novagen) to prepare an expression vector.

### Example 1-2. Preparation of CB1 point mutation and combinational variants

To improve the activity of CB1, mutation sites were selected and primers were designed to prepare 7 kinds of point mutation and combinational variants (hereinafter, referred to as M1, M2, M3, M4, M5, M6, and M7, SEQ ID NOS: 9, 10, 3, 11, 12, 13, and 14). The mutation sites based on the amino acid sequence of SEQ ID NO: 1, the amino acids after mutation, and primer sequences for preparing the variants are described in order in Table 1 below.

**[Table 1]**

| Name of variant | Mutation | | SEQ ID NO. | Primer sequence (5' →3') |
|---|---|---|---|---|
| M1 | A147S+A15 5S | Forward | 15 | GGCCCACCCAGCGTTGAGCGCTGAATCTCCTCACC ACTCAAGCGGGAATTACGGTATTTT |
| | | Reverse | 16 | AAAATACCGTAATTCCCGCTTGAGTGGTGAGGAGAT TCAGCGCTCAACGCTGGGTGGGCC |
| M2 | N90V | Forward | 17 | TTTGAATATTTGGGCACCAGCCCAAGTGTCGGGTGG GGCTC |
| | | Reverse | 18 | GAGCCCCACCCGACACTTGGGCTGGTGCCCAAATA TTCAAA |
| M3 | A135L | Forward | 19 | GTTGTTACGTTCAATTACCGCCTGGGGATCTTAGGT TTTTTGGCC |
| | | Reverse | 20 | GGCCAAAAAACCTAAGATCCCCAGGCGGTAATTGAA CGTAACAAC |
| M4 | A155S | Forward | 21 | GGCTGCTGAATCTCCTCACCACTCAAGCGGGAATTA CGGTATTTT |
| | | Reverse | 22 | AAAATACCGTAATTCCCGCTTGAGTGGTGAGGAGAT TCAGCAGCC |
| M5 | L170V | Forward | 23 | AGCCGCGTTAAGATGGGTGAAATCTAACGTAGCGG |
| | | Reverse | 24 | CCGCTACGTTAGATTTCACCCATCTTAACGCGGCT |
| M6 | V209F | Forward | 25 | GCCATTATCGGAAGGGTTGTTTCATGGGGCCATATT ACAGA |
| | | Reverse | 26 | TCTGTAATATGGCCCCATGAAACAACCCTTCCGATA ATGGC |
| M7 | T252L | Forward | 27 | GCCGACGCTGGGGAGTTACTGAAGATAGCCCAGTC ACG |
| | | Reverse | 28 | CGTGACTGGGCTATCTTCAGTAACTCCCCAGCGTCG GC |

In detail, 7 kinds of CB1 point mutation and combinational variants were prepared by PCR using, as a template, the polynucleotide encoding CB1 (SEQ ID NO: 1) prepared in Example 1-1, which was cloned into a pET vector, primers (SEQ ID NOS: 15, 16, 17, 18, 19, 20, 21, 22, 23, 24, 25, 26, 27, and 28 in Table 1), and a PCR premix (iNtRON, cat no. 25185). PCR was performed using Eppendorf Mastercycler Nexus GX2, and the reaction conditions are as follows.
Initial denaturation - 94°C, 2 min
Denaturation - 94°C, 20 sec
Annealing - 55°C, 10 sec
Extension - 68°C, 14 min (16 cycles from denaturation to extension)
Final Extension - 68°C, 5 min

The truncated variants thus prepared were ligated using a QuickChange Site-Directed Mutagenesis kit (Agilent, Cat# 200518), and then transformed into *E. coli* Dh5α strain, and the presence of sequence mutations was examined through sequencing.

### Example 1-3. Comparative assessment of activity of CB1 and variants

The expression vectors of CB1 and 7 kinds of variants prepared in Examples 1-1 and 1-2 were transformed into *E. coli* BL21 (DE3), inoculated into a sterile LB medium (BD Difco), and pre-cultured at 37°C, 200 rpm for 16 hours, respectively. Thereafter, 1/100 volume of the medium was inoculated into a flask comprising a sterile LB medium, and cultured at 37°C, 200 rpm until absorbance (OD₆₀₀) reached between 0.4 to 0.5, and then isopropyl β-D-1-thiogalactopyranoside (IPTG) was added to a final concentration of 1 mM, and further cultured for 16 hours, and then the cells were recovered by centrifugation. The recovered cells were added and resuspended in 20 ml of a lysis buffer (50 mM Tris-HCl pH 8.0, 100 mM NaCl, 10 mM imidazole), and then sonicated and centrifuged to obtain a crude enzyme solution. The crude enzyme solution was applied and adsorbed onto Ni-NTA resin (Qiagen, Cat no. 30230), and then a washing buffer (only 20 mM imidazole concentration in the lysis buffer composition) and an elution buffer (only 250 mM imidazole concentration in the lysis buffer composition) were applied in sequence to purify the enzyme.

The protein concentration was determined by mixing 5 µl of the diluted enzyme solution and 250 µl of Bradford solution (Quick Start^{™} Bradford 1x Dye Reagent, #5000205) and measuring absorbance at a wavelength of 595 nm.

To analyze the activity of purified CB1 and CB1 variants against fumonisin B1 (hereinafter, referred to as FUM), the purified enzyme reaction solution (50 mM Tris-HCl, pH 7.4) was treated with fumonisin B1 (CAS 116355-83-0, fumonisin B1 from *Fusarium moniliforme*) dissolved in a 1:1 solution of acetonitrile and distilled water, and the reaction was allowed at 25°C for 30 minutes. After sampling at 5-minute intervals, the reaction was stopped by adding 4 times the volume of methanol. The amount of fumonisin B1 in each sample was analyzed using LC-FLD, and the analysis conditions are as follows.

### - Conditions for fumonisin B1 concentration analysis (LC-FLD)

Liquid chromatography-fluorescence detection analysis was performed using Waters Acquity UPLC and a fluorescence detector. After separation from the column, analysis was performed after post-column derivatization with an o-phthalaldehyde (OPA) solution before entering the fluorescence detector, and the detailed analysis conditions are as follows.
(1) Chromatography: Waters Acquity UPLC System
(2) Column: Waters Acquity UPLC BEH C18 1.7um 2.1x150mm
(3) Column temperature: 40°C
(4) Flow rate: 0.25 mL/min
(5) Sample injection: 10.0 µL
(6) Mobile phase:
A: 13.7 mM Octanesulfonic acid + 25 mM Potassium dihydrogen phosphate in DW (pH 2.1, by H₃PO₄)
B: 13.7 mM Octanesulfonic acid + 25 mM Potassium dihydrogen phosphate in 50% Acetonitrile (pH 2.1, by H₃PO₄)

(7) Elution conditions:

**[Table 2]**

| Time (min) | %A | %B |
|---|---|---|
| 0.0 | 25 | 75 |
| 8.0 | 25 | 75 |
| 8.1 | 0 | 100 |
| 10.5 | 0 | 100 |
| 10.6 | 25 | 75 |
| 16.0 | 25 | 75 |

(8) Detection wavelength: Excitation wavelength 338 nm, Emission wavelength 425 nm
(9) Post-column derivatization method
Reagent: 5.2 mM o-phthalaldehyde (OPA) in borate buffer
Reagent flow rate: 0.25 mL/min
Reactor temperature: 40°C

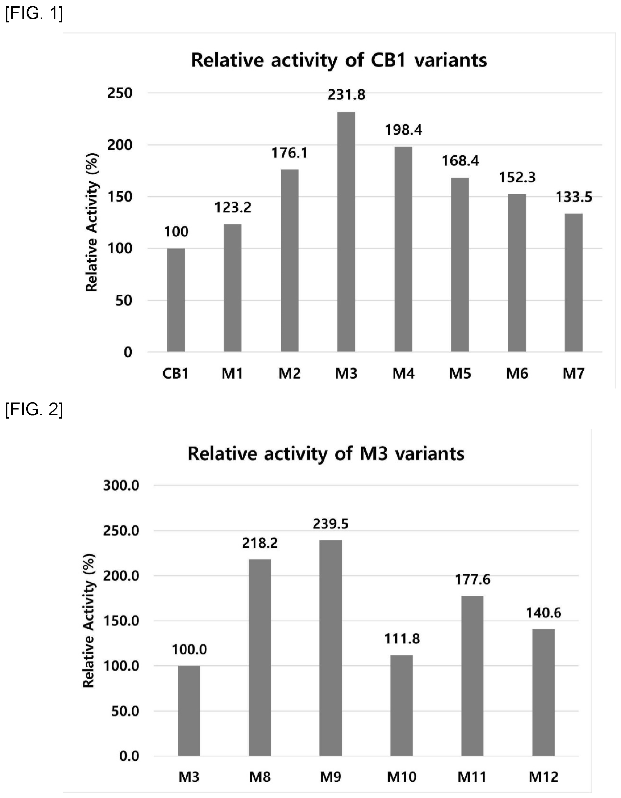

Under the above conditions, the remaining amount of FUM in the reaction product was measured. Sampling was performed at 5-minute intervals to calculate the slope value of the measured remaining amount of FUM, and the relative activity was expressed by comparing the specific activity of each enzyme. The results are shown in Table 3 and FIG. 1.

**[Table 3]**

| Variant | Slope of FUM remaining amount | Relative Activity (%) |
|---|---|---|
| CB1 | -0.049 | 100.0 |
| M1 | -0.061 | 123.2 |
| M2 | -0.087 | 176.1 |
| M3 | -0.114 | 231.8 |
| M4 | -0.098 | 198.4 |
| M5 | -0.083 | 168.4 |
| M6 | -0.075 | 152.3 |
| M7 | -0.066 | 133.5 |

As a result, it was confirmed that the activities of 7 kinds of CB1 variants (M1, M2, M3, M4, M5, M6, and M7) were improved by 123.2% to 231.8% or more compared to the template CB1. Through this, it was confirmed that the degradation activity for fumonisin B1 was improved through point mutations and combinational mutations.

### Example 2: Preparation of M3 variants (1)

### Example 2-1. Preparation of M3 point mutation and combinational variants (1)

To improve the activity of M3 prepared in Example 1-2, mutation sites were selected and primers were designed to prepare 5 kinds of point mutations and combinational variants (hereinafter, referred to as M8, M9, M10, M11, and M12, SEQ ID NOS: 29, 30, 31, 32, and 33). The mutation sites based on the amino acid sequence of SEQ ID NO: 3, the amino acids after mutation, and primer sequences for preparing the variants are described in order in Table 4 below.

**[Table 4]**

| Name of variant | Mutation | | SEQ ID NO. | Primer sequence (5' → 3') |
|---|---|---|---|---|
| M8 | N90V | Forward | 17 | TTTGAATATTTGGGCACCAGCCCAAGT GTCGGGTGGGGCTC |
| | | Reverse | 18 | GAGCCCCACCCGACACTTGGGCTGGT GCCCAAATATTCAAA |
| M9 | A147S+A15 5S | Forward | 15 | GGCCCACCCAGCGTTGAGCGCTGAAT CTCCTCACCACTCAAGCGGGAATTACG GTATTTT |
| | | Reverse | 16 | AAAATACCGTAATTCCCGCTTGAGTGG TGAGGAGATTCAGCGCTCAACGCTGG GTGGGCC |
| M10 | A155S | Forward | 21 | GGCTGCTGAATCTCCTCACCACTCAAG CGGGAATTACGGTATTTT |
| | | Reverse | 22 | AAAATACCGTAATTCCCGCTTGAGTGG TGAGGAGATTCAGCAGCC |
| M11 | L170V | Forward | 23 | AGCCGCGTTAAGATGGGTGAAATCTAA CGTAGCGG |
| | | Reverse | 24 | CCGCTACGTTAGATTTCACCCATCTTA ACGCGGCT |
| M12 | I293Q | Forward | 34 | CTTTTGCCACCGGAGAGCAGCATCGC GTCCCCCTGAT |
| | | Reverse | 35 | ATCAGGGGGACGCGATGCTGCTCTCC GGTGGCAAAAG |

In detail, 5 kinds of M3 point mutation and combinational variants were prepared by PCR using, as a template, the polynucleotide encoding M3 (SEQ ID NO: 3) prepared in Example 1-2 which was cloned into a pET vector, primers (SEQ ID NOS: 17, 18, 15, 16, 21, 22, 23, 24, 34, and 35 in Table 4), and a PCR premix (iNtRON, cat no. 25185). PCR was performed using Eppendorf Mastercycler Nexus GX2, and the reaction conditions are as follows.
Initial denaturation - 94°C, 2 min
Denaturation - 94°C, 20 sec
Annealing - 55°C, 10 sec
Extension - 68°C, 14 min (16 cycles from denaturation to extension)
Final Extension - 68°C, 5 min

The truncated variants thus prepared were ligated using a QuickChange Site-Directed Mutagenesis kit (Agilent, Cat# 200518), and then transformed into *E. coli* Dh5α strain, and the presence of sequence mutations was examined through sequencing.

### Example 2-2. Comparative assessment of activity of M3 and variants (1)

The expression vectors of M3 and 5 kinds of variants prepared in Example 1-2 were transformed into *E. coli* BL21 (DE3), inoculated into a sterile LB medium (BD Difco), and pre-cultured at 37°C, 200 rpm for 16 hours, respectively. Thereafter, 1/100 volume of the medium was inoculated into a flask comprising a sterile LB medium, and cultured at 37°C, 200 rpm until absorbance (OD₆₀₀) reached between 0.4 to 0.5, and then isopropyl β-D-1-thiogalactopyranoside (IPTG) was added to a final concentration of 1 mM, and further cultured for 16 hours, and then the cells were recovered by centrifugation. The recovered cells were added and resuspended in 20 ml of a lysis buffer (50 mM Tris-HCl pH 8.0, 100 mM NaCl, 10 mM imidazole), and then sonicated and centrifuged to obtain a crude enzyme solution. The crude enzyme solution was applied and adsorbed onto Ni-NTA resin (Qiagen, Cat no. 30230), and then a washing buffer (only 20 mM imidazole concentration in the lysis buffer composition) and an elution buffer (only 250 mM imidazole concentration in the lysis buffer composition) were applied in sequence to purify the enzyme.

The protein concentration was determined by mixing 5 µl of the diluted enzyme solution and 250 µl of Bradford solution (Quick Start^{™} Bradford 1x Dye Reagent, #5000205) and measuring the absorbance at a wavelength of 595 nm.

To analyze the activity of purified M3 and M3 variants against fumonisin B1 (hereinafter, referred to as FUM), the purified enzyme reaction solution (50 mM Tris-HCl, pH 7.4) was treated with fumonisin B1 (CAS 116355-83-0, fumonisin B1 from *Fusarium moniliforme*) dissolved in a 1:1 solution of acetonitrile and distilled water, and the reaction was allowed at 25°C for 30 minutes. After sampling at 5-minute intervals, the reaction was stopped by adding 4 times the volume of methanol. The amount of fumonisin B1 in each sample was analyzed using LC-FLD, and the analysis conditions are as follows.

### - Conditions for fumonisin B1 concentration analysis (LC-FLD)

Liquid chromatography-fluorescence detection analysis was performed using a Waters Acquity UPLC and a fluorescence detector. After separation from the column, analysis was performed after post-column derivatization with an o-phthalaldehyde (OPA) solution before entering the fluorescence detector, and the detailed analysis conditions are as follows.
(1) Chromatography: Waters Acquity UPLC System
(2) Column: Waters Acquity UPLC BEH C18 1.7 um 2.1x150 mm
(3) Column temperature: 40°C
(4) Flow rate: 0.25 mL/min
(5) Sample injection: 10.0 µL
(6) Mobile phase:
A: 13.7 mM Octanesulfonic acid + 25 mM Potassium dihydrogen phosphate in DW (pH 2.1, by H₃PO₄)
B: 13.7 mM Octanesulfonic acid + 25 mM Potassium dihydrogen phosphate in 50% Acetonitrile (pH 2.1, by H₃PO₄)

(7) Elution conditions:

**[Table 5]**

| Time(min) | %A | %B |
|---|---|---|
| 0.0 | 25 | 75 |
| 8.0 | 25 | 75 |
| 8.1 | 0 | 100 |
| 10.5 | 0 | 100 |
| 10.6 | 25 | 75 |
| 16.0 | 25 | 75 |

(8) Detection wavelength: Excitation wavelength 338 nm, Emission wavelength 425 nm
(9) Post-column derivatization method
Reagent: 5.2 mM o-phthalaldehyde (OPA) in borate buffer
Reagent flow rate: 0.25 mL/min
Reactor temperature: 40°C

Under the above conditions, the remaining amount of FUM in the reaction product was measured. Sampling was performed at 5-minute intervals to calculate the slope value of the measured remaining amount of FUM, and the relative activity was expressed by comparing the specific activity of each enzyme. The results are as shown in Table 6 and FIG. 2.

**[Table 6]**

| Variant | Slope of FUM remaining amount | Relative Activity (%) |
|---|---|---|
| M3 | -0.060 | 100.0 |
| M8 | -0.131 | 218.2 |
| M9 | -0.144 | 239.5 |
| M10 | -0.067 | 111.8 |
| M11 | -0.106 | 177.6 |
| M12 | -0.084 | 140.6 |

As a result, it was confirmed that the activities of 5 kinds of M3 variants (M8, M9, M10, M11, and M12) were improved by 111.8% to 239.5% or more, as compared to the template M3. Through this, it was confirmed that the degradation activity for fumonisin B1 was improved through point mutations and combinational mutations.

### Example 3: Preparation of M3 variants (2)

### Example 3-1. Preparation of M3 point mutation and combinational variants (2)

To improve the activity of M3, additional mutation sites were selected and primers were designed to prepare 4 kinds of point mutations and combinational variants (hereinafter, referred to as M13, M14, M15, and M16, SEQ ID NOS: 36, 37, 5, and 38). The mutation sites based on the amino acid sequence of SEQ ID NO: 3, the amino acids after mutation, and primer sequences for preparing the variants are described in order in Table 7 below.

**[Table 7]**

| Name of variant | Mutation | | SEQ ID NO. | Primer sequence (5' → 3') |
|---|---|---|---|---|
| M13 | N90A+R354 A | Forward | 39 | tttgaatatttgggcaccagcccaagcgtcgggtggggctc |
| | | Reverse | 40 | gagccccacccgacgcttgggctggtgcccaaatattcaaa |
| | | Forward | 41 | AGCCGCAGTAGCCGCCTTGTTCGGAGATA |
| | | | | GTC |
| | | Reverse | 42 | GACTATCTCCGAACAAGGCGGCTACTGCG GCT |
| M14 | A147T | Forward | 43 | gcccacccagcgttgaccgctgaatctcctcac |
| | | Reverse | 44 | gtgaggagattcagcggtcaacgctgggtgggc |
| M15 | A407G | Forward | 45 | cgagatcccttacgtgtttggcaatttgggtccttccagtg |
| | | Reverse | 46 | cactggaaggacccaaattgccaaacacgtaagggatctcg |
| M16 | V199L | Forward | 47 | gcctgcttgatatgctgctggtgtcgccatt |
| | | Reverse | 48 | aatggcgacaccagcagcatatcaagcaggc |

In detail, 4 kinds of M3 point mutation and combinational variants were prepared by PCR using, as a template, the polynucleotide encoding M3 (SEQ ID NO: 3) prepared in Example 1-2 which was cloned into a pET vector, primers (SEQ ID NOS: 39, 40, 41, 42, 43, 44, 45, 46, 47, and 48 in Table 7), and a PCR premix (iNtRON, cat no. 25185). PCR was performed using Eppendorf Mastercycler Nexus GX2, and the reaction conditions are as follows.
Initial denaturation - 94°C, 2 min
Denaturation - 94°C, 20 sec
Annealing - 55°C, 10 sec
Extension - 68°C, 14 min (16 cycles from denaturation to extension)
Final Extension - 68°C, 5 min

The truncated variants thus prepared were ligated using a QuickChange Site-Directed Mutagenesis kit (Agilent, Cat# 200518), and then transformed into *E. coli* Dh5α strain. The presence of sequence mutations was examined through sequencing.

### Example 3-2. Comparative assessment of activity of M3 and variants

The expression vectors of M3 and 4 kinds of variants prepared in Examples 1-2 and 3-1 were transformed into *E. coli* BL21 (DE3), inoculated into a sterile LB medium (BD Difco), and pre-cultured at 37°C, 200 rpm for 16 hours, respectively. Thereafter, 1/100 volume of the medium was inoculated into a flask comprising a sterile LB medium, and cultured at 37°C, 200 rpm until absorbance (OD₆₀₀) reached between 0.4 to 0.5, and then isopropyl β-D-1-thiogalactopyranoside (IPTG) was added to a final concentration of 1 mM, and further cultured for 16 hours, and then the cells were recovered by centrifugation. The recovered cells were added and resuspended in 20 ml of a lysis buffer (50 mM Tris-HCl pH 8.0, 100 mM NaCl, 10 mM imidazole), and then sonicated and centrifuged to obtain a crude enzyme solution. The crude enzyme solution was applied and adsorbed onto Ni-NTA resin (Qiagen, Cat no. 30230), and then a washing buffer (only 20 mM imidazole concentration in the lysis buffer composition) and an elution buffer (only 250 mM imidazole concentration in the lysis buffer composition) were applied in sequence to purify the enzyme.

The protein concentration was determined by mixing 5 µl of the diluted enzyme solution and 250 µl of Bradford solution (Quick Start^{™} Bradford 1x Dye Reagent, #5000205) and measuring absorbance at a wavelength of 595 nm.

To analyze the activity of purified M3 and M3 variants for fumonisin B1 (hereinafter, referred to as FUM), the purified enzyme reaction solution (50 mM Tris-HCl, pH 7.4) was treated with fumonisin B1 (CAS 116355-83-0, fumonisin B1 from *Fusarium moniliforme*) dissolved in a 1:1 solution of acetonitrile and distilled water, and the reaction was allowed at 25°C for 15 minutes, and then the reaction was stopped by adding 4 times the volume of methanol. The amount of fumonisin B1 in each sample was analyzed using LC-FLD, and the analysis conditions are as follows.

### - Conditions for fumonisin B1 concentration analysis (LC-FLD)

Liquid chromatography-fluorescence detection analysis was performed using Waters Acquity UPLC and a fluorescence detector. After separation from the column, analysis was performed after post-column derivatization with an o-phthalaldehyde (OPA) solution before entering the fluorescence detector, and the detailed analysis conditions are as follows.
(1) Chromatography: Waters Acquity UPLC System
(2) Column: Waters Acquity UPLC BEH C18 1.7 um 2.1x150 mm
(3) Column temperature: 40°C
(4) Flow rate: 0.25 mL/min
(5) Sample injection: 10.0 µL
(6) Mobile phase:
A: 13.7 mM Octanesulfonic acid + 25 mM Potassium dihydrogen phosphate in DW (pH 2.1, by H₃PO₄)
B: 13.7 mM Octanesulfonic acid + 25 mM Potassium dihydrogen phosphate in 50% Acetonitrile (pH 2.1, by H₃PO₄)

(7) Elution conditions:

**[Table 8]**

| Time(min) | %A | %B |
|---|---|---|
| 0.0 | 25 | 75 |
| 8.0 | 25 | 75 |
| 8.1 | 0 | 100 |
| 10.5 | 0 | 100 |
| 10.6 | 25 | 75 |
| 16.0 | 25 | 75 |

(8) Detection wavelength: Excitation wavelength 338 nm, Emission wavelength 425 nm
(9) Post-column derivatization method
Reagent: 5.2 mM o-phthalaldehyde (OPA) in borate buffer
Reagent flow rate: 0.25 mL/min
Reactor temperature: 40°C

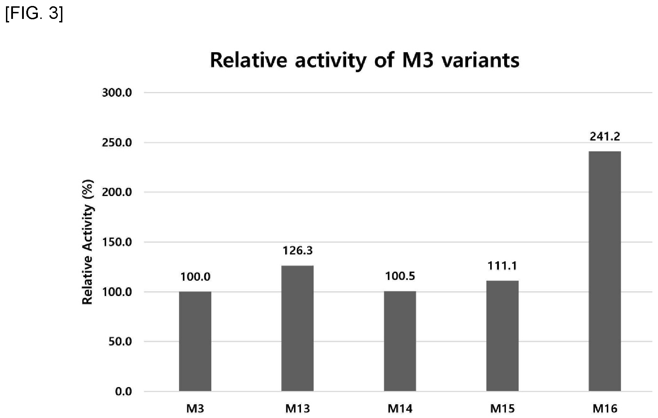

Under the above conditions, the remaining amount of FUM in the reaction product was measured, and the relative activities of the variants were measured using the ratio of the total degraded FUM, and the results are as shown in Table 9 and FIG. 3.

**[Table 9]**

| Variant | FUM Remaining (%) | FUM Degradation (%) | Relative Activity (%) |
|---|---|---|---|
| M3 | 86.4 | 13.6 | 100.0 |
| M13 | 82.9 | 17.1 | 126.3 |
| M14 | 86.3 | 13.7 | 100.5 |
| M15 | 84.9 | 15.1 | 111.1 |
| M16 | 67.2 | 32.8 | 241.2 |

As a result, when the enzymatic activity was expressed as a relative ratio, the activities of 4 kinds of M3 variants (M13, M14, M15, and M16) were confirmed to be improved by 100.5% to 241.2% or more compared to the template M3. Through this, it was confirmed that the degradation activity for fumonisin B1 was improved through point mutations and combinational mutations.

### Example 4: Preparation of M15 variants

### Example 4-1. Preparation of M15 point mutation and combinational variants

To improve the activity of M15, additional mutation sites were selected and primers were designed to prepare 5 kinds of point mutation and combinational variants (hereinafter, referred to as M17, M18, M19, M20, and M21, SEQ ID NOS: 7, 49, 50, 51, and 52). The mutation sites based on the amino acid sequence of SEQ ID NO: 5, amino acids after mutation, and primer sequences for preparing the variants are described in order in Table 10 below.

**[Table 10]**

| Name of variant | Mutation | | SEQ ID NO. | Primer sequence (5' → 3') |
|---|---|---|---|---|
| M17 | G29A | Forward | 53 | gggtaccgttcgcggcacctcccat |
| | | Reverse | 54 | atgggaggtgccgcgaacggtaccc |
| M18 | M452G | Forward | 55 | ggatcctaacgggggtggccttcctcactggccgg |
| | | Reverse | 56 | ccggccagtgaggaaggccacccccgttaggatcc |
| M19 | A147T | Forward | 43 | gcccacccagcgttgaccgctgaatctcctcac |
| | | Reverse | 44 | gtgaggagattcagcggtcaacgctgggtgggc |
| M20 | T290A | Forward | 57 | tactgctgcttttgccgcgggagagattcatcgcg |
| | | Reverse | 58 | cgcgatgaatctctcccgcggcaaaagcagcagta |
| M21 | V199L | Forward | 47 | gcctgcttgatatgctgctggtgtcgccatt |
| | | Reverse | 48 | aatggcgacaccagcagcatatcaagcaggc |

In detail, 5 kinds of M15 point mutation and combinational variants were prepared by PCR using, as a template, the polynucleotide encoding M15 (SEQ ID NO: 5) prepared in Example 3-1 which was cloned into a pET vector, primers (SEQ ID NOS: 53, 54, 55, 56, 43, 44, 57, 58, 47, and 48 in Table 10), and a PCR premix (iNtRON, cat no. 25185). PCR was performed using Eppendorf Mastercycler Nexus GX2, and the reaction conditions are as follows.
Initial denaturation - 94°C, 2 min
Denaturation - 94°C, 20 sec
Annealing - 55°C, 10 sec
Extension - 68°C, 14 min (16 cycles from denaturation to extension)
Final Extension - 68°C, 5 min

The truncated variants thus prepared were ligated using a QuickChange Site-Directed Mutagenesis kit (Agilent, Cat# 200518), and then transformed into *E. coli* Dh5α strain, and the presence of sequence mutations was examined through sequencing.

### Example 4-2. Comparative assessment of activity of M15 and variants

The expression vectors of M15 and 5 kinds of variants prepared in Examples 3-1 and 4-1 were transformed into *E. coli* BL21 (DE3), inoculated into a sterile LB medium (BD Difco), and pre-cultured at 37°C, 200 rpm for 16 hours, respectively. Thereafter, 1/100 volume of the medium was inoculated into a flask comprising a sterile LB medium, and cultured at 37°C, 200 rpm until absorbance (OD₆₀₀) reached between 0.4 to 0.5, and then isopropyl β-D-1-thiogalactopyranoside (IPTG) was added to a final concentration of 1 mM, and further cultured for 16 hours, and then the cells were recovered by centrifugation. The recovered cells were added and resuspended in 20 ml of a lysis buffer (50 mM Tris-HCl pH 8.0, 100 mM NaCl, 10 mM imidazole), and then sonicated and centrifuged to obtain a crude enzyme solution. The crude enzyme solution was applied and adsorbed onto Ni-NTA resin (Qiagen, Cat no. 30230), and then a washing buffer (only 20 mM imidazole concentration in the lysis buffer composition) and an elution buffer (only 250 mM imidazole concentration in the lysis buffer composition) were applied in sequence to purify the enzyme.

The protein concentration was determined by mixing 5 µl of the diluted enzyme solution and 250 µl of Bradford solution (Quick Start^{™} Bradford 1x Dye Reagent, #5000205) and measuring absorbance at a wavelength of 595 nm.

To analyze the activity of purified M15 and M15 variants for fumonisin B1 (hereinafter, referred to as FUM), the purified enzyme reaction solution (50 mM Tris-HCl, pH 7.4) was treated with fumonisin B1 (CAS 116355-83-0, fumonisin B1 from *Fusarium moniliforme*) dissolved in a 1:1 solution of acetonitrile and distilled water, and the reaction was allowed at 25°C for 15 minutes, and then the reaction was stopped by adding 4 times the volume of methanol. The amount of fumonisin B1 in each sample was analyzed using LC-FLD, and the analysis conditions are as follows.

### - Conditions for fumonisin B1 concentration analysis (LC-FLD)

Liquid chromatography-fluorescence detection analysis was performed using Waters Acquity UPLC and a fluorescence detector. After separation from the column, analysis was performed after post-column derivatization with an o-phthalaldehyde (OPA) solution before entering the fluorescence detector, and the detailed analysis conditions are as follows.
(1) Chromatography: Waters Acquity UPLC System
(2) Column: Waters Acquity UPLC BEH C18 1.7 um 2.1x150 mm
(3) Column temperature: 40°C
(4) Flow rate: 0.25 mL/min
(5) Sample injection: 10.0 µL
(6) Mobile phase:
A: 13.7 mM Octanesulfonic acid + 25 mM Potassium dihydrogen phosphate in DW (pH 2.1, by H₃PO₄)
B: 13.7 mM Octanesulfonic acid + 25 mM Potassium dihydrogen phosphate in 50% Acetonitrile (pH 2.1, by H₃PO₄)

(7) Elution conditions:

**[Table 11]**

| Time(min) | %A | %B |
|---|---|---|
| 0.0 | 25 | 75 |
| 8.0 | 25 | 75 |
| 8.1 | 0 | 100 |
| 10.5 | 0 | 100 |
| 10.6 | 25 | 75 |
| 16.0 | 25 | 75 |

(8) Detection wavelength: Excitation wavelength 338 nm, Emission wavelength 425 nm
(9) Post-column derivatization method
Reagent: 5.2 mM o-phthalaldehyde (OPA) in borate buffer
Reagent flow rate: 0.25 mL/min
Reactor temperature: 40°C

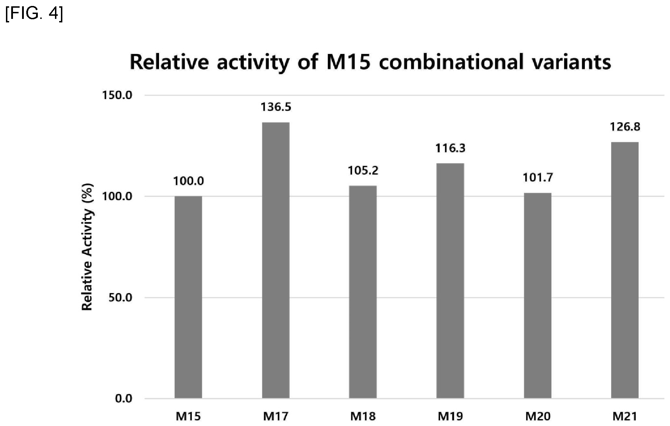

Under the above conditions, the remaining amount of FUM in the reaction product was measured, and the relative activities of the variants were measured using the ratio of the total degraded FUM, and the results are as shown in Table 12 and FIG. 4.

**[Table 12]**

| Variant | FUM Remaining (%) | FUM Degradation (%) | Relative Activity (%) |
|---|---|---|---|
| M15 | 42.8 | 57.2 | 100 |
| M17 | 21.9 | 78.1 | 136.5 |
| M18 | 39.8 | 60.2 | 105.2 |
| M19 | 33.5 | 66.5 | 116.3 |
| M20 | 41.9 | 58.1 | 101.7 |
| M21 | 27.5 | 72.5 | 126.8 |

As a result, when the enzymatic activity was expressed as a relative ratio, the activities of 5 kinds of M15 variants (M17, M18, M19, M20, and M21) were confirmed to be improved by 101.7% to 136.5% or more, as compared to the template M15. Through this, it was confirmed that the degradation activity for fumonisin B1 was improved through point mutations and combinational mutations.

### Example 5: Preparation of M17 variants

### Example 5-1. Preparation of M17 point mutation and combinational variants

To improve the activity of M17, additional mutation sites were selected, and primers were designed to prepare 2 kinds of point mutations and combinational variants (hereinafter, referred to as M22 and M23, SEQ ID NOS: 59 and 60). The mutation sites based on the amino acid sequence of SEQ ID NO: 7, the amino acids after mutation, and primer sequences for preparing the variants are described in order in Table 13 below.

**[Table 13]**

| Name of variant | Mutation | | SEQ ID NO. | Primer Sequence (5' → 3') |
|---|---|---|---|---|
| M22 | M452G | Forward | 55 | ggatcctaacgggggtggccttcctcactggccgg |
| | | Reverse | 56 | ccggccagtgaggaaggccacccccgttaggatcc |
| M23 | V199L | Forward | 47 | gcctgcttgatatgctgctggtgtcgccatt |
| | | Reverse | 48 | aatggcgacaccagcagcatatcaagcaggc |

In detail, 2 kinds of M17 point mutations and combinational variants were prepared by PCR using, as a template, the polynucleotide encoding M17 (SEQ ID NO: 7) prepared in Example 4-1 which was cloned into a pET vector, primers (SEQ ID NOS: 57, 58, 48, and 49 in Table 13), and a PCR premix (iNtRON, cat no. 25185). PCR was performed using Eppendorf Mastercycler Nexus GX2, and the reaction conditions are as follows.
Initial denaturation - 94°C, 2 min
Denaturation - 94°C, 20 sec
Annealing - 55°C, 10 sec
Extension - 68°C, 14 min (16 cycles from denaturation to extension)
Final Extension - 68°C, 5 min

The truncated variants thus prepared were ligated using a QuickChange Site-Directed Mutagenesis kit (Agilent, Cat# 200518), and then transformed into *E. coli* Dh5α strain. The presence of sequence mutations was examined through sequencing.

### Example 5-2. Comparative assessment of activity of M17 and variants

The expression vectors of M17 and 2 kinds of variants prepared in Examples 4-1 and 5-1 were transformed into *E. coli* BL21 (DE3), inoculated into a sterile LB medium (BD Difco), and pre-cultured at 37°C, 200 rpm for 16 hours, respectively. Thereafter, 1/100 volume of the medium was inoculated into a flask comprising a sterile LB medium, and cultured at 37°C, 200 rpm until absorbance (OD₆₀₀) reached between 0.4 to 0.5, and then isopropyl β-D-1-thiogalactopyranoside (IPTG) was added to a final concentration of 1 mM, and further cultured for 16 hours, and then the cells were recovered by centrifugation. The recovered cells were added and resuspended in 20 ml of a lysis buffer (50 mM Tris-HCl pH 8.0, 100 mM NaCl, 10 mM imidazole), and then sonicated and centrifuged to obtain a crude enzyme solution. The crude enzyme solution was applied and adsorbed onto Ni-NTA resin (Qiagen, Cat no. 30230), and then a washing buffer (only 20 mM imidazole concentration in the lysis buffer composition) and an elution buffer (only 250 mM imidazole concentration in the lysis buffer composition) were applied in sequence to purify the enzyme.

The protein concentration was determined by mixing 5 µl of the diluted enzyme solution and 250 µl of Bradford solution (Quick Start^{™} Bradford 1x Dye Reagent, #5000205) and measuring the absorbance at a wavelength of 595 nm.

To analyze the activity of purified M17 and M17 variants against fumonisin B1 (hereinafter, referred to as FUM), the purified enzyme reaction solution (50 mM Tris-HCl, pH 7.4) was treated with fumonisin B1 (CAS 116355-83-0, fumonisin B1 from *Fusarium moniliforme*) dissolved in a 1:1 solution of acetonitrile and distilled water, and the reaction was allowed at 25°C for 15 minutes, and then the reaction was stopped by adding 4 times the volume of methanol. The amount of fumonisin B1 in each sample was analyzed using LC-FLD, and the analysis conditions are as follows.

### - Conditions for fumonisin B1 concentration analysis (LC-FLD)

Liquid chromatography-fluorescence detection analysis was performed using Waters Acquity UPLC and a fluorescence detector. After separation from the column, analysis was performed after post-column derivatization with an o-phthalaldehyde (OPA) solution before entering the fluorescence detector, and the detailed analysis conditions are as follows.
(1) Chromatography: Waters Acquity UPLC System
(2) Column: Waters Acquity UPLC BEH C18 1.7 um 2.1x150 mm
(3) Column temperature: 40°C
(4) Flow rate: 0.25 mL/min
(5) Sample injection: 10.0 µL
(6) Mobile phase:
A: 13.7 mM Octanesulfonic acid + 25 mM Potassium dihydrogen phosphate in DW (pH 2.1, by H₃PO₄)
B: 13.7 mM Octanesulfonic acid + 25 mM Potassium dihydrogen phosphate in 50% Acetonitrile (pH 2.1, by H₃PO₄)

(7) Elution conditions:

**[Table 14]**

| Time(min) | %A | %B |
|---|---|---|
| 0.0 | 25 | 75 |
| 8.0 | 25 | 75 |
| 8.1 | 0 | 100 |
| 10.5 | 0 | 100 |
| 10.6 | 25 | 75 |
| 16.0 | 25 | 75 |

(8) Detection wavelength: Excitation wavelength 338 nm, Emission wavelength 425 nm
(9) Post-column derivatization method
Reagent: 5.2 mM o-phthalaldehyde (OPA) in borate buffer
Reagent flow rate: 0.25 mL/min
Reactor temperature: 40°C

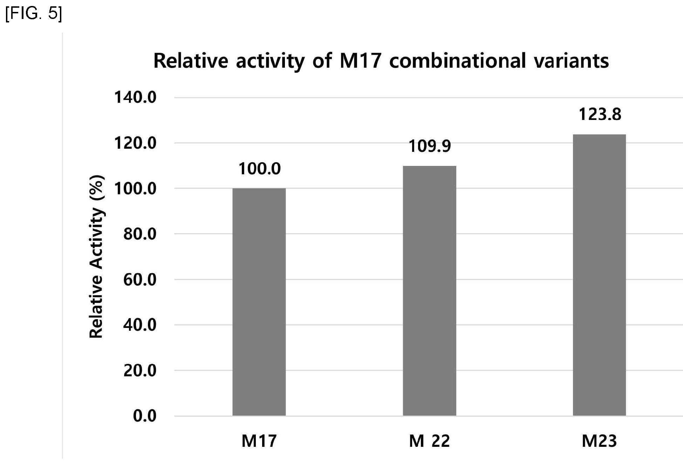

Under the above conditions, the remaining amount of FUM in the reaction product was measured, and the relative activities of the variants were measured using the ratio of the total degraded FUM, and the results are as shown in Table 15 and FIG. 5.

**[Table 15]**

| Variant | FUM Remaining (%) | FUM Degradation (%) | Relative Activity (%) |
|---|---|---|---|
| M17 | 39.9 | 60.1 | 100 |
| M22 | 34.0 | 66.0 | 109.9 |
| M23 | 25.6 | 74.4 | 123.8 |

As a result, when the enzymatic activity was expressed as a relative ratio, the activities of 2 kinds of M17 variants (M22 and M23) were confirmed to be improved by 109.9% to 123.8% or more, as compared to the template M17. Through this, it was confirmed that the degradation activity for fumonisin B1 was improved through point mutations and combinational mutations.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A variant polypeptide having fumonisin degradation activity,
wherein
i) the variant polypeptide has 70% or more and less than 100% sequence identity to SEQ ID NO: 1; and/or
ii) the variant polypeptide is a polypeptide encoded by a polynucleotide having 70% or more and less than 100% sequence identity to a sequence encoding a mature polypeptide of SEQ ID NO: 1; and/or
iii) the variant polypeptide is a polypeptide encoded by a polynucleotide that hybridizes to (a) a mature polypeptide coding sequence of SEQ ID NO: 1, (b) a cDNA thereof, or (c) the full-length complement of (a) or (b) under low stringency conditions, medium stringency conditions, medium-high stringency conditions, high stringency conditions, or very high stringency conditions; and/or
iv) the variant polypeptide is a functional fragment of the i), ii) or iii) polypeptide having fumonisin degradation activity; and
the variant polypeptide comprising any one selected from the following modifications:
deletion of amino acids, insertion of amino acids, substitution with different amino acids at one or more positions of positions 29, 90, 135, 147, 155, 170, 199, 209, 252, 290, 293, 354, 407, and 452, and combinations thereof;
where the position number is a position corresponding to the position of the polypeptide of SEQ ID NO: 1.

2. The variant polypeptide of claim 1, wherein in the variant polypeptide having fumonisin degradation activity before modification, the amino acid at position 29 is G; the amino acid at position 90 is N; the amino acid at position 135 is A; the amino acid at position 147 is A; the amino acid at position 155 is A; the amino acid at position 170 is L; the amino acid at position 199 is V; the amino acid at position 209 is V; the amino acid at position 252 is T; the amino acid at position 290 is T; the amino acid at position 293 is I; the amino acid at position 354 is R; the amino acid at position 407 is A; and/or the amino acid at position 452 is M.

3. The variant polypeptide of claim 1, wherein the variant polypeptide comprises amino acid substitutions at one or more amino acid positions 29, 90, 135, 147, 155, 170, 199, 209, 252, 290, 293, 354, 407, and 452,
where the position number is a position corresponding to the position of the polypeptide of SEQ ID NO: 1.

4. The variant polypeptide of claim 1, wherein the variant polypeptide comprises any one or more substitutions of the following i) to xiv):
i) a substitution of the amino acid at position 29 with alanine (A);
ii) a substitution of the amino acid at position 90 with valine (V) or alanine (A);
iii) a substitution of the amino acid at position 135 with leucine (L);
iv) a substitution of the amino acid at position 147 with serine (S) or threonine (T);
v) a substitution of the amino acid at position 155 with serine (S);
vi) a substitution of the amino acid at position 170 with valine (V);
vii) a substitution of the amino acid at position 199 with leucine (L);
viii) a substitution of the amino acid at position 209 with phenylalanine (F);
ix) a substitution of the amino acid at position 252 with leucine (L);
x) a substitution of the amino acid at position 290 with alanine (A);
xi) a substitution of the amino acid at position 293 with glutamine (Q);
xii) a substitution of the amino acid at position 354 with alanine (A);
xiii) a substitution of the amino acid at position 407 with glycine (G); and
xiv) a substitution of the amino acid at position 452 with glycine(G);
where the position number is a position corresponding to the position of the polypeptide of SEQ ID NO: 1.

5. The variant polypeptide of claim 1, wherein the variant polypeptide comprises any one or more selected from the following modifications:
A147S+A155S;
N90V;
A135L;
A155S;
L170V;
V209F;
T252L;
A135L+N90V;
A135L+A147S+A155S;
A135L+A155S;
A135L+L170V;
A135L+I293Q;
A135L+N90A+R354A;
A135L+A147T;
A135L+A407G;
A135L+V199L;
A135L+A407G+G29A;
A135L+A407G+M452G;
A135L+A407G+A147T;
A135L+A407G+T290A;
A135L+A407G+V199L;
A135L+A407G+G29A+M452G;
A135L+A407G+G29A+V199L
where the position number is a position corresponding to the position of the polypeptide of SEQ ID NO: 1.

6. The variant polypeptide of claim 1, wherein the variant polypeptide has any one or more modified properties of the following i) and ii), as compared to the polypeptide consisting of the amino acid sequence of SEQ ID NO: 1:
i) increased enzymatic activity; and
ii) increased specific activity.

7. A composition comprising any one or more of the variant polypeptide of any one of claims 1 to 6; and a host cell expressing the variant polypeptide.

8. A composition for degrading fumonisin, the composition comprising any one or more of the variant polypeptide of any one of claims 1 to 6; and a host cell expressing the variant polypeptide.

9. A composition for being added to feeds, the composition comprising any one or more of the variant polypeptide of any one of claims 1 to 6; and a host cell expressing the variant polypeptide.

10. A composition for detoxifying fumonisin present in foods, feeds, or both of them, the composition comprising any one or more of the variant polypeptide of any one of claims 1 to 6; and a host cell expressing the variant polypeptide.

11. A method of degrading fumonisin, the method comprising the step of reacting fumonisin with any one or more of the variant polypeptide of any one of claims 1 to 6; and a host cell expressing the variant polypeptide.

12. A polynucleotide encoding the variant polypeptide of any one of claims 1 to 6.

13. A host cell comprising any one or more of the variant polypeptide of any one of claims 1 to 6; and the polynucleotide of claim 11.

14. A method of preparing a variant polypeptide having fumonisin degradation activity, the method comprising the steps of:
culturing the host cell of claim 13; and
recovering the variant polypeptide having fumonisin degradation activity of any one of claims 1 to 6, which is expressed in the culturing step.

15. Use of any one or more of the variant polypeptide of any one of claims 1 to 6; and a host cell expressing the variant polypeptide in degrading fumonisin.
